# EUROPÄISCHE PATENTSCHRIFT

(11) **EP 1 194 118 B1**
(45) Veröffentlichungstag und Bekanntmachung des Hinweises auf die Patenterteilung: **15.09.2004**
(21) Anmeldenummer: 01931507.6
(22) Anmeldetag: 09.03.2001
(51) Int. Cl.: A61K 7/13

(54) **MITTEL ZUR FÄRBUNG VON FASERN**
AGENT FOR COLORING FIBERS
AGENT PERMETTANT DE COLORER DES FIBRES

(30) Priorität: 10.05.2000 DE 10022743
(43) Veröffentlichungstag der Anmeldung: 10.04.2002
(73) Patentinhaber: Wella Aktiengesellschaft, 64274 Darmstadt (DE)
(72) Erfinder: JAVET, Manuela, CH-1723 Marly (CH); MUELLER, Catherine, CH-1723 Marly (CH)
(86) Internationale Anmeldenummer: PCT/EP2001/002684
(87) Internationale Veröffentlichungsnummer: WO 2001/085111

(56) Entgegenhaltungen:
- EP-A- 0 847 749
- DE-A- 19 856 342
- FR-A- 2 787 707
- GB-A- 1 528 590

## Beschreibung

Gegenstand der vorliegenden Erfindung ist ein Mittel zur Färbung von Fasern, insbesondere von menschlichen Haaren, wobei -falls gewünscht- die erhaltene Färbung zu einem beliebigen späteren Zeitpunkt auch wieder schonend entfärbt werden kann.

Haarfärbemittel werden je nach zu färbender Ausgangshaarfarbe und gewünschtem Endresultat hauptsächlich in den Bereich der Oxidationsfärbemittel oder der Tönungen unterteilt. Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen und ermöglichen eine Grauabdeckung von etwa 20 %.

Im allgemeinen waschen sich direktziehende und oxidative Tönungen nach einigen Haarwäschen heraus. Die Zeitdauer hängt u. a. sehr stark von der Haarstruktur und der verwendeten Nuance ab. Oxidative Farben können teilweise mit der Zeit verblassen, verbleiben aber in der Regel bis zum nächsten Haarschnitt im Haar. Eine jederzeit mögliche Entfernung der Haarfärbung kann jedoch dann wünschenswert sein, wenn man eine besondere Farbe nur für einen bestimmten Zeitraum tragen will, oder eine Färbung dem Anwender nicht gefällt. Ebenso kann im Falle der Haarfärbung bei Erstverwendem die Möglichkeit einer schonenden und vollständigen Entfernung der Färbung die Angst vor einer zu drastischen Farbveränderung vermindern ("Färbung auf Probe").

Aus der DE-OS 197 17 281 ist die Verwendung einer Kombination von Benzylidenketonen und Aminen und/oder Hydroxyverbindungen und/oder CH-aciden Verbindungen zur Färbung von Haaren ohne Zusatz von Oxidationsmitteln bekannt. Ebenfalls ist es aus der DE-GM 299 08 464 bekannt, dass Haare durch eine Kombination von bestimmten 1,2,3,3-Tetramethyl-3H-indoliumsalzen und Carbonylverbindungen auch ohne den Zusatz von Oxidationsmitteln dauerhaft gefärbt werden können.

Aufgabe der vorliegenden Erfindung ist es, ein Färbemittel zur Verfügung zu stellen, das eine sehr hohe Lagerstabilität aufweist und sowohl auch ohne die Verwendung eines Oxidationsmittels eine schonende und dauerhafte Färbung der Fasern als auch eine schonende und vollständige Entfernung dieser Färbung zu jedem beliebigen Zeitpunkt ermöglicht.

Überraschenderweise wurde nunmehr gefunden, dass diese Aufgabe durch die Verwendung eines durch Vermischen einer ein Enamin enthaltenden Komponente mit einer eine Carbonylverbindung und ein primäres Amin enthaltenden Komponente erhältlichen Mittels gelöst werden kann.

Gegenstand der vorliegenden Erfindung ist daher ein Mittel zur Färbung von Fasern (A), wie zum Beispiel Wolle, Seide, Baumwolle oder Haaren und insbesondere menschlichen Haaren, welches durch Vermischen zweier Komponenten (A1) und (A2) hergestellt wird und dadurch gekennzeichnet ist, dass die Komponente (A1) einen sauren pH-Wert aufweist und mindestens ein Enamin der Formel (I) oder dessen Säureadditionssalz der Formel (Ia) enthält, 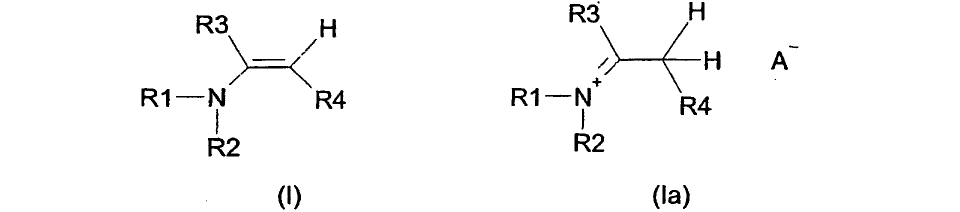 wobei gilt: **R1** gleich einem ein- oder mehrkemigen aromatischen Rest, insbesondere einem unsubstituierten 5- gliedrigen oder 6-gliedrigen Arylrest (vorzugsweise einem Phenylrest), einem unsubstituierten 5-gliedrigen oder 6-gliedrigen Heterozyklus (vorzugsweise einem Pyridylrest oder Naphthylrest), einem mit einer C1- bis C4-Alkylgruppe, einer C1- bis C4-Hydroxyalkylgruppe, einer Hydroxygruppe, einer Methoxygruppe, einer Dialkylaminogruppe oder einer Halogengruppe (F, Cl, Br, J) substituierten 5-gliedrigen oder 6-gliedrigen Arylrest (vorzugsweise einem subsituierten Phenylrest), oder einem mit einer C1- bis C4-Alkylgruppe, einer C1-bis C4-Hydroxyalkylgruppe, einer Hydroxygruppe, einer Methoxygruppe, einer Dialkylaminogruppe oder einer Halogengruppe (F, Cl, Br, J)substituierten 5- gliedrigen oder 6-gliedrigen Heterozyklus (vorzugsweise einem substituierten Pyridylrest oder einem Naphthylrest); **R2** gleich einer geradkettigen oder verzweigten C1- bis C8-Alkylgruppe, einer geradkettigen oder verzweigten C1- bis C8-Hydroxyalkylgruppe oder einer C1- bis C8-Alkoxyalkylgruppe, wobei jeweils zwischen den C-Atomen der Alkylkette Sauerstoffatome sitzen können; **R3** gleich einer geradkettigen oder verzweigten C1- bis C8-Alkylgruppe, einer C1- bis C8-Alkoxyalkylgruppe, einer geradkettigen oder verzweigten C1- bis C8-Alkylengruppe, einer C1- bis C8-Alkoxyalkylengruppe, einem Sauerstoffatom, einem Schwefelatom, einer -NH-Gruppe oder einer -NR-Gruppe, mit R gleich einer Alkylgruppe, einer Alkoxyalkylgruppe, einer Hydroxyalkylgruppe oder Wasserstoff; wobei die Reste **R1** und **R3** gemeinsam mit dem Stickstoffatom und dem Kohlenstoffatom der Enamingrundstruktur eine zyklische Verbindung bilden können, und **R4** gleich Wasserstoff, einer geradkettigen C1- bis C4-Alkylgruppe oder einer verzweigten C1- bis C4-Alkylgruppe; **A**^{**-**} gleich dem Anion einer organischen oder anorganischen Säure ist;
und die Komponente (A2) einen alkalischen pH-Wert aufweist und mindestens eine Carbonylverbindung und mindestens ein primäres Amin enthält.

Bevorzugt sind Verbindungen der Formel (I), in denen die Reste **R1** und **R3** gemeinsam mit dem Stickstoffatom und dem Kohlenstoffatom der Enamingrundstruktur eine zyklische Verbindung bilden, wobei vorzugsweise **R3** am aromatischen Rest **R1** mit dem Kohlenstoff verbunden ist, der in ortho-Stellung zum Enamin-substituierten Kohlenstoff steht.

Besonders bevorzugt sind die folgenden Enamine der Formeln (II) bis (IX), in denen **X** gleich einem mit zwei C1- bis C4-Alkylgruppen, welche gleich oder verschieden sein können, (insbesondere 2 Methylgruppen) substituierten C-Atom, einem mit einer C1- bis C4-Alkylgruppe und einer Hydroxygruppe substituierten C-Atom, einem Schwefelatom, einem alkylierten Stickstoffatom, einem nichtalkylierten Stickstoffatom oder einem Sauerstoffatom ist; und **R2** gleich einer geradkettigen oder verzweigten C1- bis C8-Alkylgruppe, einer geradkettigen oder verzweigten C1- bis C8-Hydroxyalkylgruppe, oder einer C1- bis C8-Alkoxyalkylgruppe ist, wobei jeweils zwischen den C-Atomen der Alkylkette Sauerstoffatome sitzen können; **R4** gleich Wasserstoff, einer geradkettigen C1- bis C4-Alkylgruppe oder einer verzweigten C1- bis C4-Alkylgruppe ist;
**R5, R6, R7** und **R8** unabhängig voneinander gleich Wasserstoff, einer geradkettigen oder verzweigten C1- bis C4-Alkylgruppe, einer geradkettigen oder verzweigten C1- bis C4-Hydroxyalkylgruppe, einer Hydroxygruppe, einer Methoxygruppe, einer Aminogruppe, einer Monoalkylaminogruppe, einer Dialkylaminogruppe, einer Benzylgruppe oder einem Halogenatom (F, Cl, Br, J) sind; und **A**^{**-**} gleich Chlorid, Bromid, Jodid, Sulfat, Hydrogensulfat, Toluolsulfonat, Benzolsulfonat, Monomethylsulfat, Hexafluorphosphat, Hexafluorantimonat, Tetrafluorborat, Tetraphenylborat, Formiat, Acetat oder Propionat ist, wobei das Chloridion, das Tetrafluoroboration, das Acetation und das Hydrogensulfation besonders bevorzugt sind.

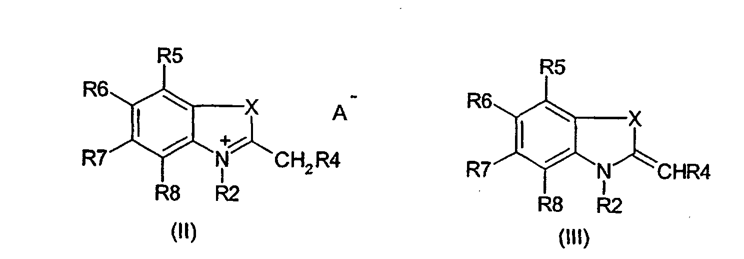

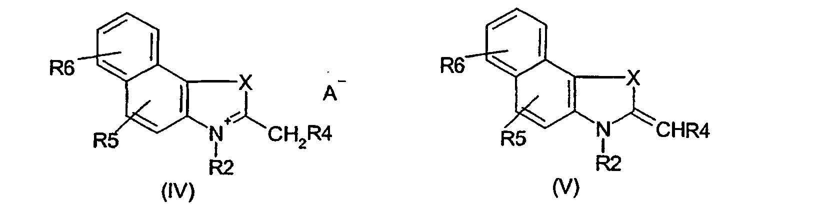

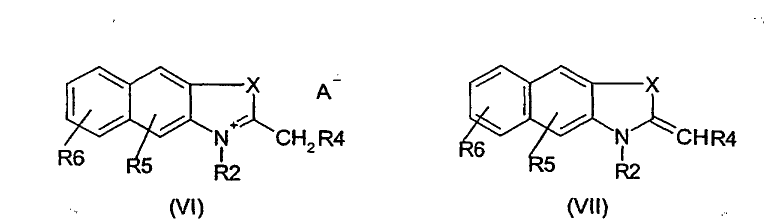

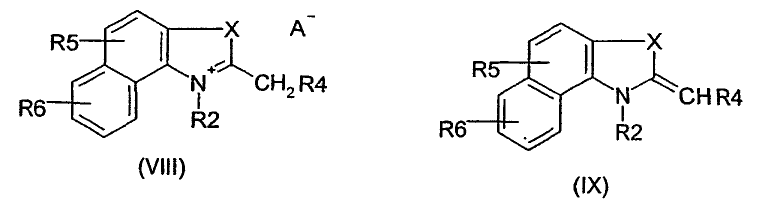

Unter den Verbindungen der Formeln (II) bis (IX) sind insbesondere die folgenden Verbindungen zu nennen: 1,3,3-Trimethyl-2-methylen-indolin sowie dessen Salze, 1,3,3,4-Tetramethyl-2-methylen-indolin sowie dessen Salze, 1,3,3,5-Tetramethyl-2-methylen-indolin sowie dessen Salze, 1,3,3,6-Tetramethyl-2-methylen-indolin sowie dessen Salze, 1,3,3,7-Tetramethyl-2-methylen-indolin sowie dessen Salze, 1,3,3,6,7-Pentamethyl-2-methylen-indolin sowie dessen Salze, 1,3,3,5,7-Pentamethyl-2-methylen-indolin sowie dessen Salze, 1,3,3,4,7-Pentamethyl-2-methylen-indolin sowie dessen Salze, 5-Chloro-1,3,3-trimethyl-2-methylen-indolin sowie dessen Salze, 5-Fluoro-1,3,3-trimethyl-2-methylen-indolin sowie dessen Salze, 5-Isopropyl-1,3,3-trimethyl-2-methylen-indolin sowie dessen Salze, 5-Hydroxy-1,3,3-trimethyl-2-methylen-indolin sowie dessen Salze, 5-Methoxy-1,3,3-trimethyl-2-methylen-indolin sowie dessen Salze, 5-Amino-1,3,3-trimethyl-2-methylen-indolin sowie dessen Salze, 6-Hydroxy-1,3,3-trimethyl-2-methylen-indolin sowie dessen Salze, 6-Methoxy-1,3,3-trimethyl-2-methylen-indolin sowie dessen Salze 5-Methoxy-6-amino-1,3,3-trimethyl-2-methylen-indolin sowie dessen Salze, 5,6-Dihydroxy-1,3,3-trimethyl-2-methylen-indolin sowie dessen Salze, 5,6-Dimethoxy-1,3,3-trimethyl-2-methylen-indolin sowie dessen Salze, 4,5-Dihydroxy-1,3,3-trimethyl-2-methylen-indolin sowie dessen Salze, 5,7-Dihydroxy-1,3,3-trimethyl-2-methylen-indolin sowie dessen Salze, 5-Amino-6-methoxy-1,3,3-trimethyl-2-methylen-indolin sowie dessen Salze, 5-Amino-7-hydroxy-1,3,3-trimethyl-2-methylen-indolin sowie dessen Salze, 5-Hydroxy-7-amino-1,3,3-trimethyl-2-methylenindolin sowie dessen Salze, 1-(2'-Hydroxyethyl)-3,3-dimethyl-2-methylenindolin sowie dessen Salze, 1,3,3-Trimethyl-2-methylen-3H-benz[e]indol sowie dessen Salze und N-Ethyl-2-methylen-benzthiazol sowie dessen Salze; wobei das 1,3,3-Trimethyl-2-methylen-indolin, 1,2,3,3-Tetramethyl-3H-indolium-chlorid, 1,2,3,3-Tetramethyl-3H-indolium-bromid, 1,2,3,3-Tetramethyl-3H-indolium-jodid, 1,2,3,3-Tetramethyl-3H-indolium-sulfat, 1,2,3,3-Tetramethyl-3H-indolium-hydrogensulfat, 1,2,3,3-Tetramethyl-3H-indolium-methylsulfat, 1,2,3,3-Tetramethyl-3H-indolium-hexafluorophosphat, 1,2,3,3-Tetramethyl-3H-indolium-hexafluoro-antimonat, 1,2,3,3-Tetramethyl-3H-indolium-tetrafluoroborat, 1,2,3,3,5-Pentamethyl-3H-indolium-jodid, 1,2,3,3,7-Pentamethyl-3H-indolium-tetrafluoroborat, 1,2,3,3,6,7-Hexamethyl-3H-indolium-tetrafluoroborat, 1,2,3,3,5,7-Hexamethyl-3H-indolium-tetrafluoroborat, 1,2,3,3,4,7-Hexamethyl-3H-indolium-tetrafluoroborat, 5-Chloro-1,2,3,3-tetramethyl-3H-indolium-jodid, 5-Fluoro-1,2,3,3-tetramethyl-3H-indolium-jodid, 5-Isopropyl-1,2,3,3-tetramethyl-3H-indolium-jodid, 5-Methoxy-1,2,3,3-tetramethyl-3H-indolium-jodid, 5-Hydroxy-1,2,3,3-tetramethyl-3H-indolium-jodid, 1,2,3,3-Tetramethyl-3H-benz[e]indolium-chlorid, 1,2,3,3-Tetramethyl-3H-benz[e]indolium-bromid, 1,2,3,3-Tetramethyl-3H-benz[e]indolium-jodid, 1,2,3,3-Tetramethyl-3H-benz[e]indolium-sulfat, 1,2,3,3-Tetramethyl-3H-benz[e]indolium-hexafluorphosphat, 1,2,3,3-Tetramethyl-3H-benz[e]indolium-methylsulfat, 1,2,3,3-Tetramethyl-3H-benz[e]indolium-hexafluorantimonat, 1,2,3,3-Tetramethyl-3H-benz[e]indolium-tetrafluorborat, 1,2-Dimethyl-benzthiazolium-jodid und N-Ethyl-2-methyl-benzthiazolium-jodid besonders bevorzugt sind.

Als für die Verwendung in der Komponente (A2) geeignete Carbonylverbindungen sind insbesondere die folgenden Aldehyde zu nennen: 4-Hydroxy-3-methoxybenzaldehyd (Vanillin), 3-Hydroxy-4-methoxy-benzaldehyd (Isovanillin), 3,4-Dihydroxy-benzaldehyd, 4-Hydroxybenzaldehyd, 3,5-Dimethoxy-4-hydroxy-benzaldehyd, 4-Dimethylaminobenzaldehyd, 4-Methyl-5-imidazol-carboxaldehyd, 4- Dimethylamino-zimtaldehyd, 4-Hydroxy-2-methoxy-benzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphth-aldehyd, 4-Methoxy-1-naphthaldehyd, 4-Dimethylamino-1-naphthaldehyd, 4'-Hydroxy-biphenyl-1-carbaldehyd, 2-Hydroxy-3-methoxybenzaldehyd, 2,4-Dihydroxybenzaldehyd, 3,4-Dihydroxy-benzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxy-benzaldehyd, 2,5-Dimethoxy-benzaldehyd, 3,5-Dimethoxybenzaldehyd, 3,4-Dimethoxybenzaldehyd, Indol-3-carbaldehyd, Benzol-1,4-dicarbaldehyd, 4-Ethoxybenzaldehyd, 2-Methyl-1,4-naphthochinon, 4-Carboxybenzaldehyd, 4-Hydroxy-3-methoxyzimtaldehyd, 3,5-Dimethoxy-4-hydroxy-zimtaldehyd, 3-Methoxy-4-(1-pyrrolidinyl)-benzaldehyd, 4-Diethylamino-3-methoxybenzaldehyd, 1,2-Phthaldialdehyd, Pyrrol-2-aldehyd, Thiophen-2-aldehyd, Thiophen-3-aldehyd, Chromone-3-carboxaldehyd, 6-Methyl-4-oxo-1 (4H)-benzopyran-3-carbaldehyd, N-Methylpyrrol-2-aldehyd, 5-Methylfurfural, 6-Hydroxychromen-3-carboxaldehyd, 6-Methylindol-3-carboxaldehyd, 4-Dibutylaminobenzaldehyd, N-Ethylcarbazol-3-aldehyd, 4-Diethylamino-2-hydroxybenzaldehyd, 3,4-Dimethoxy-5-hydroxybenzaldehyd, 5-(4-(Diethylamino)phenyl)-2,4-pentadienal, 2,3-Thiophendicarboxaldehyd, 2,5-Thiophendicarboxaldehyd, 2-Methoxy-1-naphthaldehyd, 3-Ethoxy-4-hydroxybenzaldehyd, 2-Nitrobenzaldehyd, 3-Nitrobenzaldehyd und 4-Nitrobenzaldehyd.

Als für die Verwendung in der Komponente (A2) geeignete primären Amine sind insbesondere die folgenden Verbindungen zu nennen: Alkanolamine, wie zum Beispiel Monoethanolamin, 1-Amino-2-propanol oder 3-Amino-1-propanol, oder aromatische Amine, wie zum Beispiel 1,4-Diamino-benzol, 1,4-Diamino-2-methyl-benzol, 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylamino-anilin, 4-Dimethylamino-anilin, 4-Diethylamino-anilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4 aminophenyl)(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methy(phenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol, 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 3-[(2-Hydroxyethyl)amino]-anilin, 3-[(2-Aminoethyl)amino]-anilin, 1,3-Diamino-2,4-dimethoxy-benzol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 5-Amino-2-ethyl-phenol, 2-Amino-3-hydroxy-pyridin, 5-Amino-4-chlor-2-methyl-phenol, 3,4-Diamino-benzoesäure, 6-Amino-3,4-dihydro-1,4(2H)-benzoxazin oder deren Salze, oder Aminosäuren, wie zum Beispiel Arginin, Lysin, Tyrosin, Histidin, Phenylalanin oder Tryptophan.

Die Enamine der Formeln (I) bis (IX), die Carbonylverbindungen sowie die primären Amine sind in der gebrauchsfertigen Komponente (A) jeweils in einer Gesamtmenge von etwa 0,01 bis 10 Gewichtsprozent, vorzugsweise etwa 0,1 bis 5 Gewichtsprozent, enthalten.

Die Zubereitungsform für die Komponenten (A1) und (A2) sowie des gebrauchsfertigen Färbemittels (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Enamine der Formel (I) beziehungsweise der Carbonylverbindungen/Amine mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, femer Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Weiterhin kann das erfindungsgemäße Färbemittel gegebenenfalls zusätzlich übliche, physiologisch unbedenkliche, direktziehende Farbstoffe aus der Gruppe der Nitrofarbstoffe, Azofarbstoffe, Chinonfarbstoffe und Triphenylmethanfarbstoffe enthalten.

Diese direktziehenden Farbstoffe können in der Komponente (A1) und/oder der Komponente (A2) jeweils in einer Gesamtmenge von etwa 0,02 bis 20 Gewichtsprozent, vorzugsweise 0,2 bis 10 Gewichtsprozent, eingesetzt werden, wobei die Gesamtmenge an direktziehenden Farbstoffen in dem durch Vermischen der Komponenten (A1) und (A2) erhaltenen gebrauchsfertigen Färbemittel etwa 0,01 bis 10 Gewichtsprozent, vorzugsweise 0,1 bis 5 Gewichtsprozent, beträgt.

Der pH-Wert des gebrauchsfertigen Färbemittels (A) beträgt in der Regel 3 bis 12, vorzugsweise etwa 6 bis 11, wobei sich der pH-Wert des gebrauchsfertigen Färbemittels (A) bei der Mischung der sauer eingestellten Komponente (A1) mit der alkalisch eingestellten Komponente (A2) auf einen Wert einstellt, der durch die Säuremenge in der Komponente (A1) und die Alkalimenge in der Komponente (A2) sowie durch das Mischungsverhältnis dieser beiden Komponenten beinflußt wird.

Die Komponente (A1) weist vorzugsweise einen pH-Wert von etwa 1 bis 4,5, insbesondere 1,5 bis 3, auf, während die Komponente (A2) vorzugsweise einen pH-Wert von etwa 7,5 bis 12, insbesondere 8 bis 11, aufweist.

Zur Einstellung des für die Färbung gewünschten pH-Wertes der Komponenten (A1) und (A2) sowie des gebrauchsfertigen Färbemittels (A) können -falls erforderlich- alkalisierende Mittel, wie zum Beispiel Alkanolamine, Alkylamine, Alkalihydroxide oder Ammoniumhydroxid und Alkalicarbonate oder Ammoniumcarbonate, oder Säuren, wie zum Beispiel Milchsäure, Essigsäure, Weinsäure, Phosphorsäure, Salzsäure, Zitronensäure, Ascorbinsäure oder Borsäure, verwendet werden.

Das gebrauchsfertige Färbemittel wird unmittelbar vor der Anwendung durch Vermischen der Komponenten (A1) und (A2) hergestellt und sodann auf die Faser, insbesondere menschliche Haare, aufgetragen. Je nach gewünschter Farbtiefe läßt man diese Mischung 5 bis 60 Minuten, vorzugsweise 15 bis 30 Minuten, bei einer Temperatur von etwa 20 bis 50 °C, insbesondere bei 30 bis 40 °C einwirken. Anschließend wird die Faser mit Wasser gespült und gegebenenfalls mit einem Shampoo gewaschen.

Ein weiterer Gegenstand der vorliegenden Erfindung ist ein Mehrkomponenten-Kit, bestehend aus einem Mittel der Komponente (A1), einem Mittel der Komponente (A2), sowie gegebenenfalls einem Mittel zur Einstellung des pH-Wertes. Selbstverständlich können auch die Mittel der Komponenten (A1) und (A2) aus mehreren Einzelkomponenten bestehen, welche erst unmittelbar vor der Anwendung miteinander vermischt werden. Besonders bevorzugt ist jedoch ein 2-Komponenten-Kit, bestehend aus einem Mittel der Komponente (A1) und einem Mittel der Komponente (A2).

Das erfindungsgemässe Färbemittel ist sehr lagerstabil und ermöglicht eine schonende, gleichmässige und dauerhafte Färbung der Fasern, insbesondere von Keratinfasem, wie zum Beispiel menschlichen Haaren, wobei die erhaltene Färbung nicht nachdunkelt. Überraschenderweise können diese Färbungen zu einem beliebigen Zeitpunkt durch Sulfite auf einfache und schonende Weise wieder entfernt werden.

Ein weiterer Gegenstand der vorliegenden Erfindung ist daher ein Verfahren zum temporären Färben von Fasern, bei dem die Faser zunächst mit dem erfindungsgemässen Färbemittel (A) gefärbt wird, und die Färbung zu einem beliebigen späteren Zeitpunkt mit einem als entfärbendes Agenz mindestens ein Sulfit enthaltenden Entfärbemittel wieder entfernt wird; sowie ein Mehrkomponenten-Kit zum Färben und späteren Entfärben von Fasern, enthaltend das erfindungsgemässe Färbemittel (A) sowie ein sulfithaltiges Entfärbemittel (B).

Als Sulfit können beispelsweise Ammoniumsulfit, Alkalisulfite oder Erdalkalisulfite verwendet werden, wobei Natriumsulfit und Ammoniumsulfit besonders bevorzugt sind. Die Gesamtmenge an Sulfiten in dem Entfärbemittel beträgt etwa 0,1 bis 10 Gewichtsprozent, vorzugsweise 2 bis 5 Gewichtsprozent.

Das Entfärbemittel kann als wässrige oder wässrig-alkoholische Lösung, als Gel, Creme, Emulsion oder Schaum vorliegen, wobei das Entfärbemittel sowohl in Form eines Einkomponentenpräparats als auch in Form eines Mehrkomponentenpräparates konfektioniert sein kann. Das Entfärbemittel kann neben der Pulverform zum Schutz vor Staubbildung auch als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranulat beziehungsweise der Brausetablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 3 bis 8, insbesondere 4 bis 7. Erforderlichenfalls kann der gewünschte pH-Wert durch Zugabe von geeigneten Säuren, beispielsweise α-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Alkanolaminen, Alkylaminen, Alkalihydroxiden, Ammoniumhydroxid, Alkalicarbonaten, Ammoniumcarbonaten oder Alkaliphosphaten, eingestellt werden.

Zur Entfärbung der mit dem erfindungsgemässen Mittel gefärbten Faser wird das Entfärbemittel auf die Faser aufgetragen und bei etwa 20 bis 50 °C für einen Zeitraum von etwa 5 bis 60 Minuten, insbesondere 15 bis 30 Minuten, einwirken gelassen. Nach Beendigung der Einwirkungszeit des Entfärbemittels wird das Haar mit Wasser gespült, gegebenenfalls mit einem Shampoo gewaschen und abschliessend getrocknet.

Die nachfolgenden Beispiele sollen den Gegenstand näher erläutern, ohne ihn auf diese Beispiele zu beschränken.

### Beispiele

### Beispiele 1.1 bis 1.35: Haarfärbemittel

| **Komponente (A1)** | |
|---|---|
| Verbindung der Formel (I) | gemäß Tabelle 1 |
| 6-O-Palmitoyl-L-ascorbinsäure | 0,3 g |
| Cetylstearylalkohol | 12,0 g |
| Laurylethersulfat, 28%ige wässrige Lösung | 10,0 g |
| Ethanol | 23,0 g |
| Wasser, vollentsalzt | ad 100,0 g |

Der Cetylstearylalkohol-wird bei 80 °C geschmolzen. Das Laurylethersulfat und 95 % des Wassers werden auf 80 °C erhitzt und zum geschmolzenen Cetylstearylalkohol gegeben und gerührt bis sich eine Creme ergibt. Bei Raumtemperatur wird die Verbindung der Formel (I) zusammen mit dem Ethanol, dem restlichen Wasser und der 6-O-Palmitoyl-L-ascorbinsäure zugegeben.

| **Komponente (A2)** | |
|---|---|
| Aldehydverbindung | gemäß Tabelle 1 |
| primäres Amin | gemäß Tabelle 1 |
| Cetylstearylalkohol | 12,0 g |
| Laurylethersulfat, 28%ige wässrige Lösung | 10,0 g |
| Ethanol | 23,0 g |
| Wasser, vollentsalzt | ad 100,0 g |

Der Cetylstearylalkohol wird bei 80 °C geschmolzen. Das Laurylethersulfat und 95 % des Wassers werden auf 80 °C erhitzt und zum geschmolzenen Cetylstearylalkohol gegeben und gerührt bis sich eine Creme ergibt. Bei Raumtemperatur wird der Aldehyd zusammen mit dem Ethanol und dem restlichen Wasser zugegeben. Der pH-Wert der Creme wird mit dem jeweiligen primären Amin, gegebenenfalls unter Zusatz von 10 %iger Natronlauge, auf den jeweils in Tabelle 1 angegebenen pH-Wert eingestellt.

Die Komponente (A1) und die Komponente (A2) werden im Verhältnis 1:1 miteinander vermischt. Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.
Das Haar kann zu jedem beliebigen Zeitpunkt (beispielsweise nach mehreren Tagen oder Wochen) mit einer 5 %igen Natriumsulfit-Lösung (pH = 5) innerhalb von 20 Minuten bei 40 °C wieder vollständig entfärbt werden.
Die Färbe- und Entfärbeergebnisse sind in der nachfolgenden Tabelle 1 zusammengefasst.

Die in den vorliegenden Beispielen angegebenen L*a*b*-Farbmesswerte wurden mit einem Farbmessgerät der Firma Minolta, Typ Chromameter II, ermittelt. Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

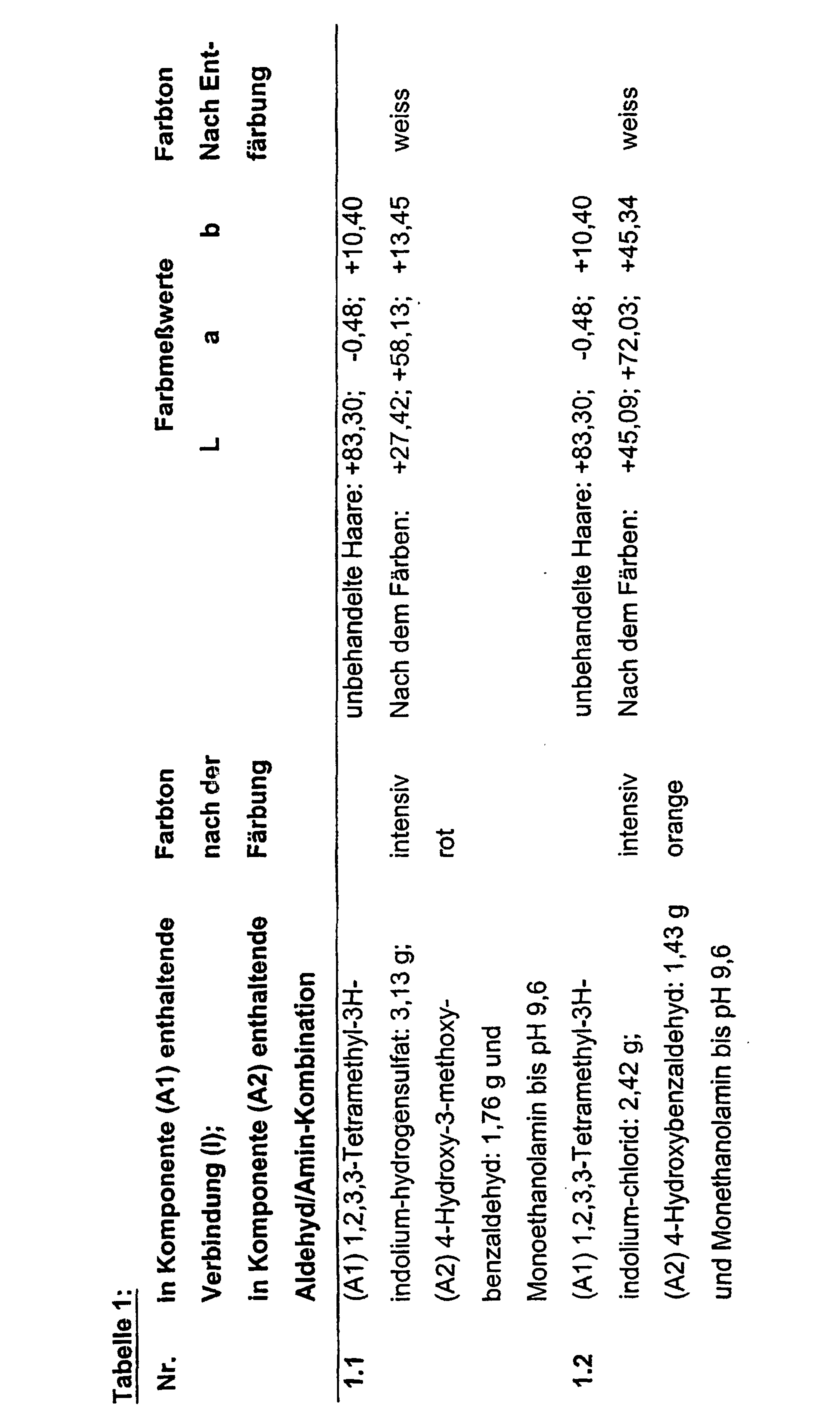

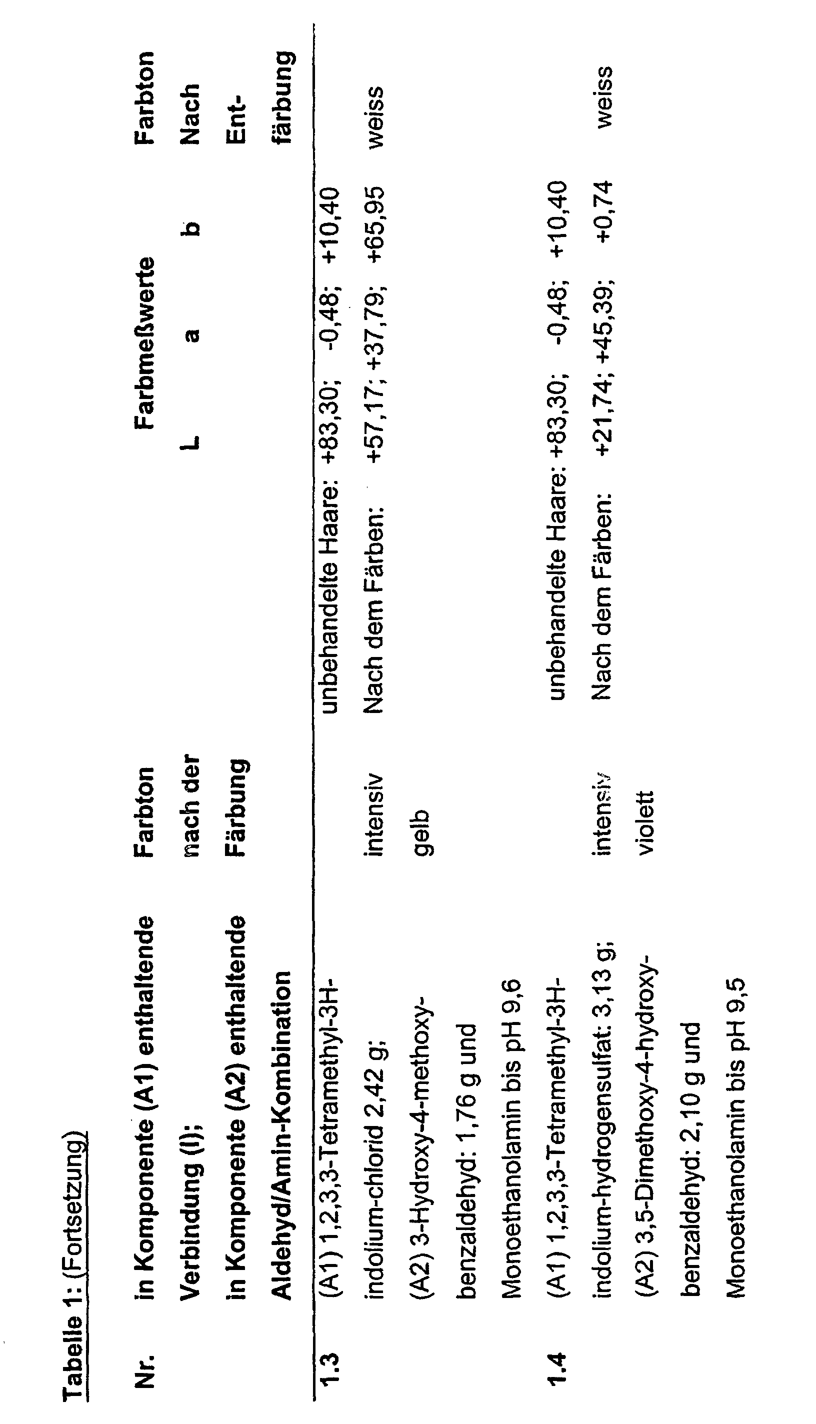

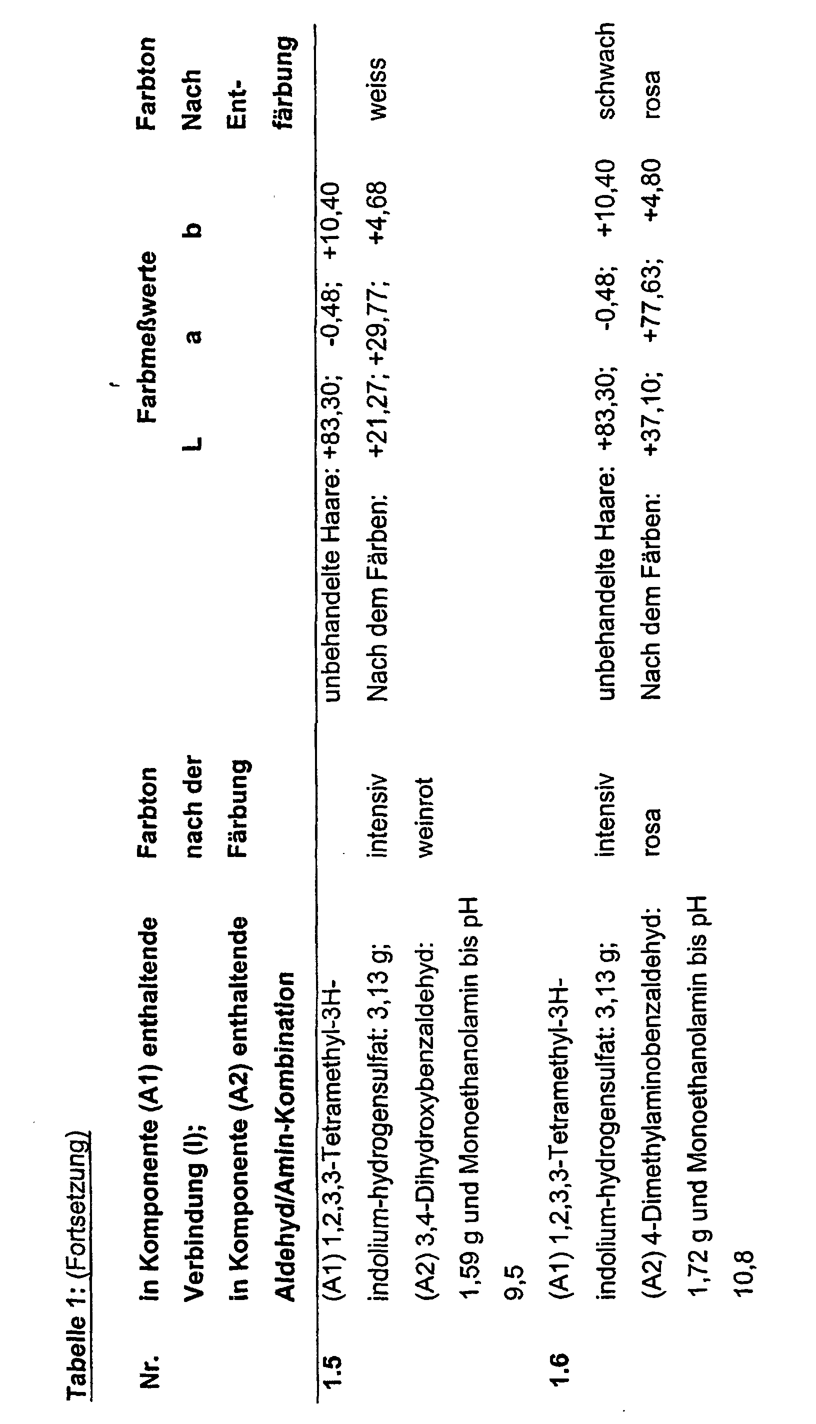

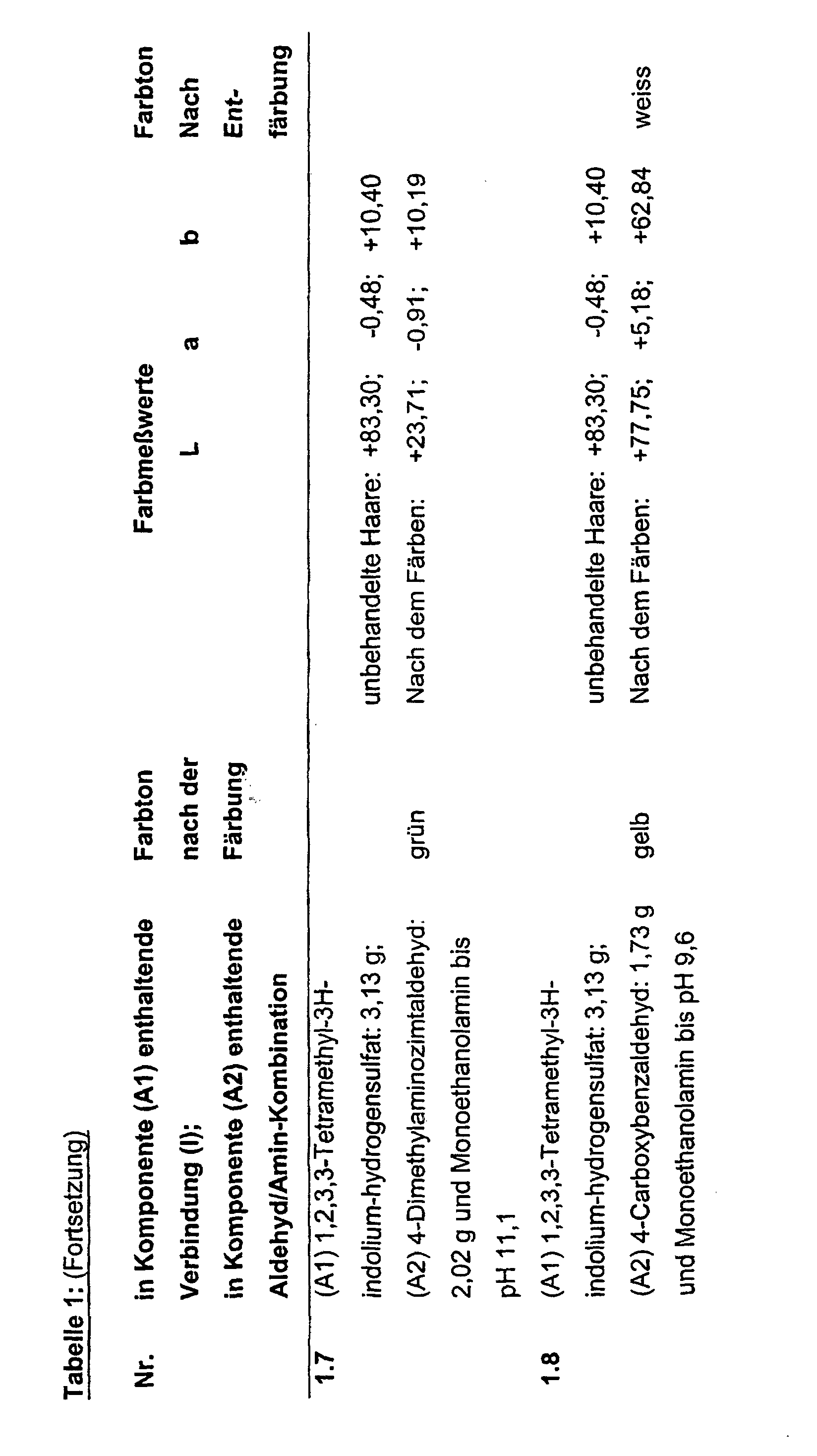

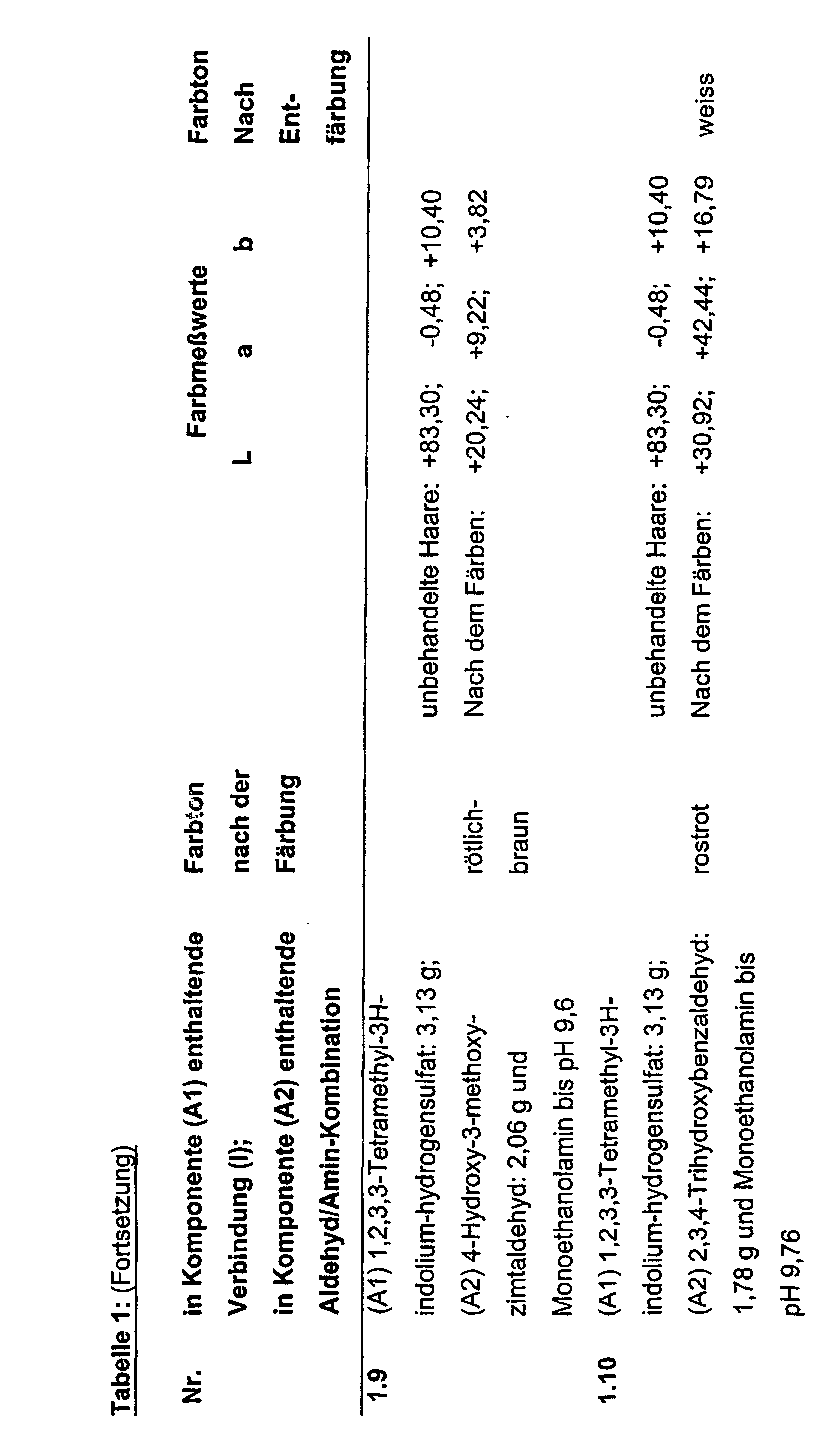

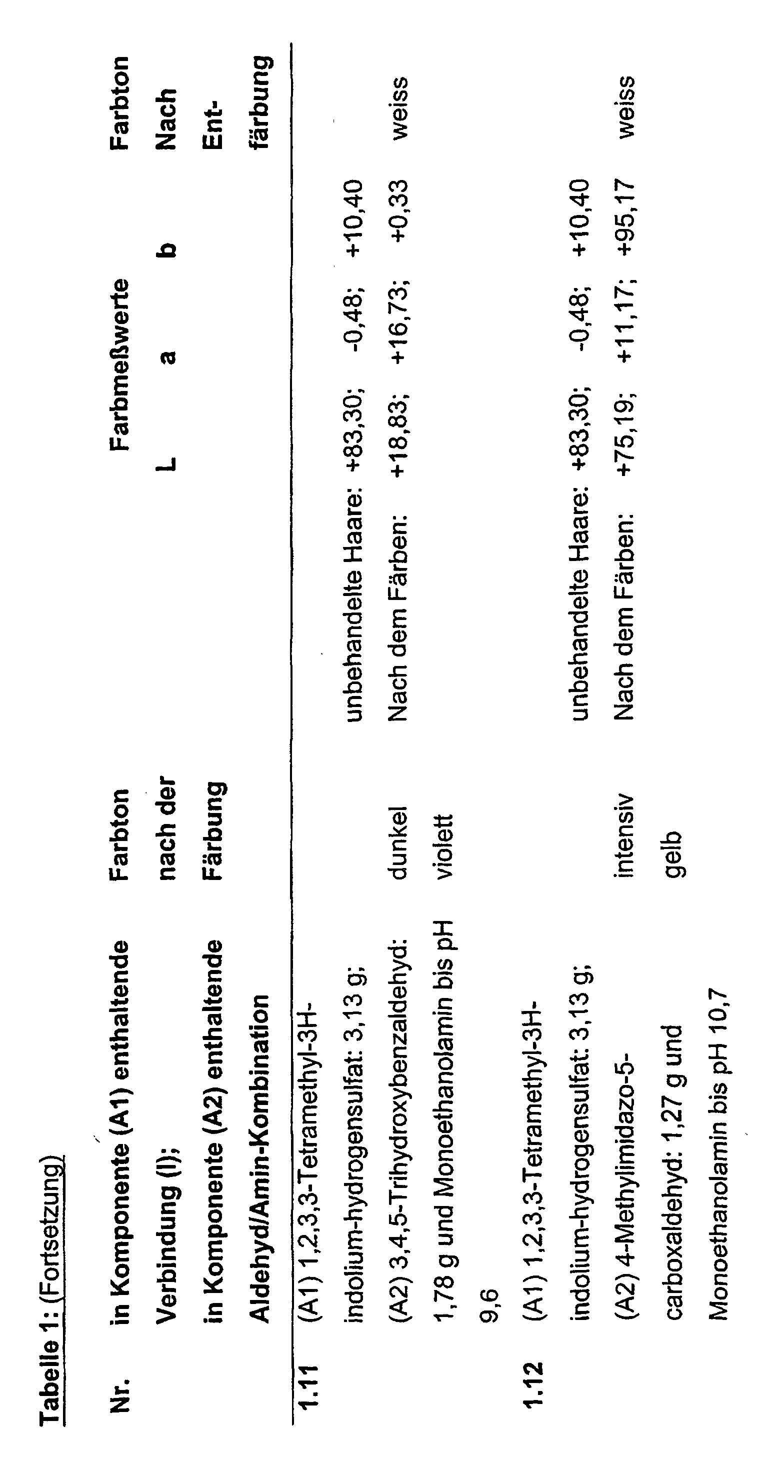

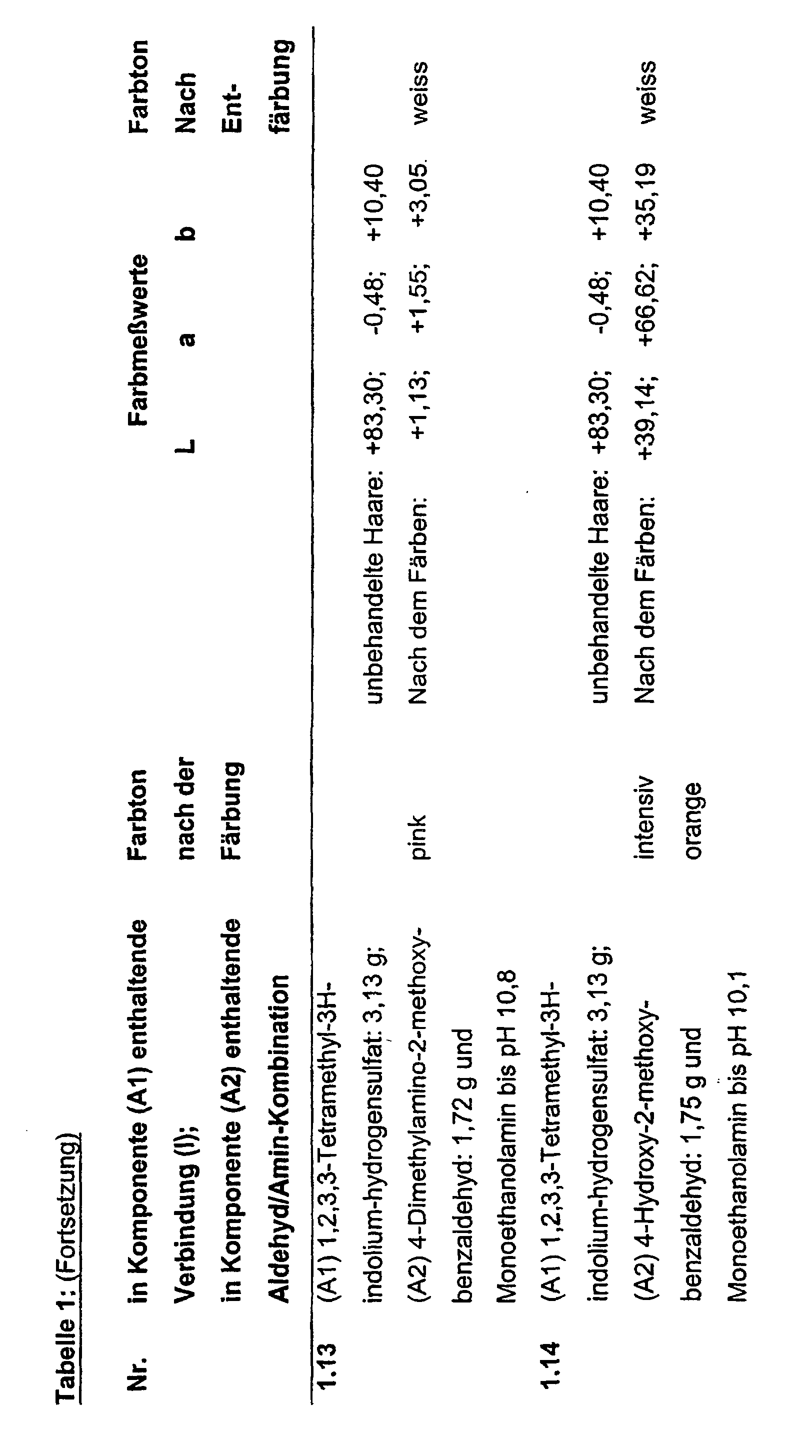

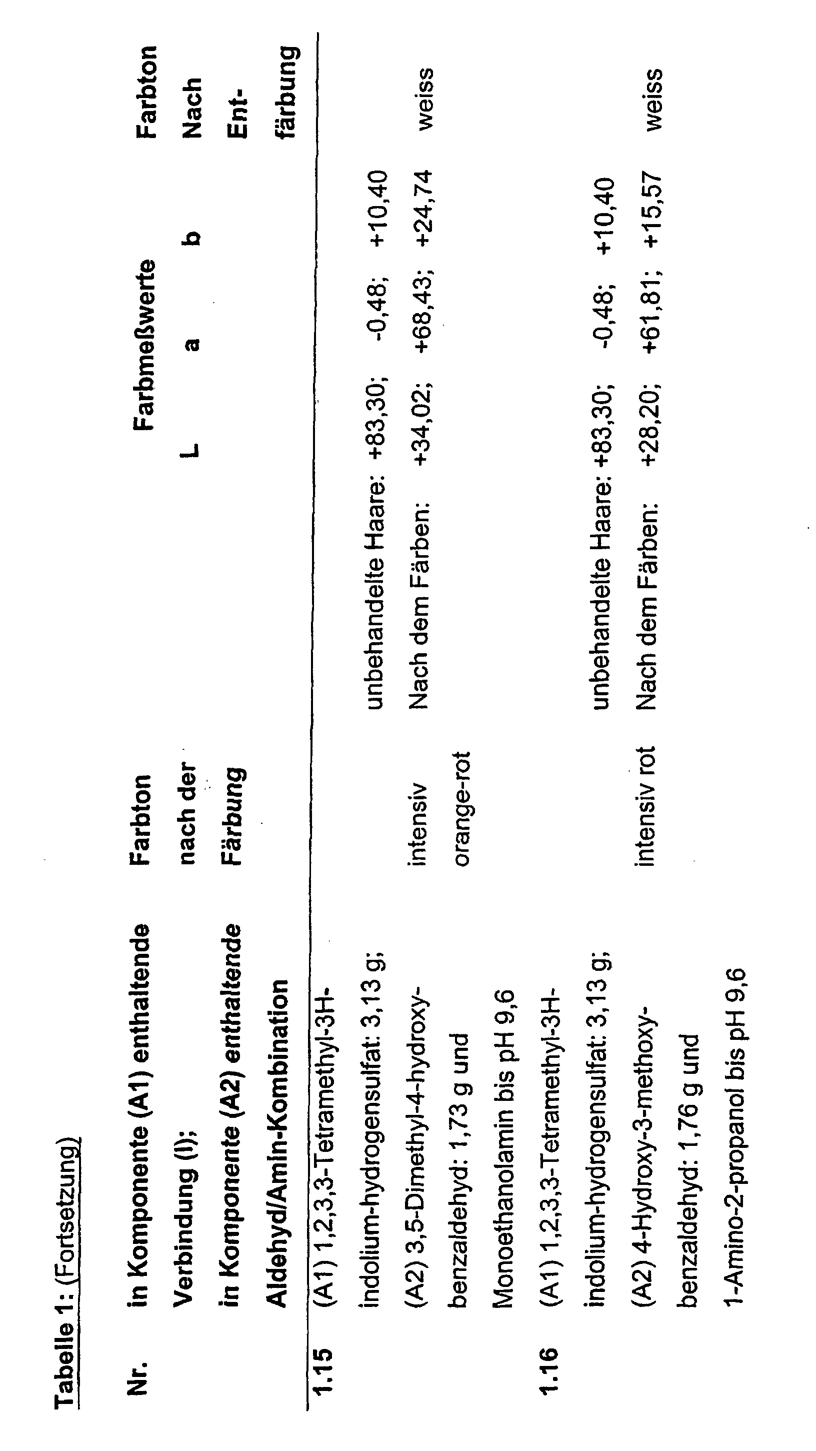

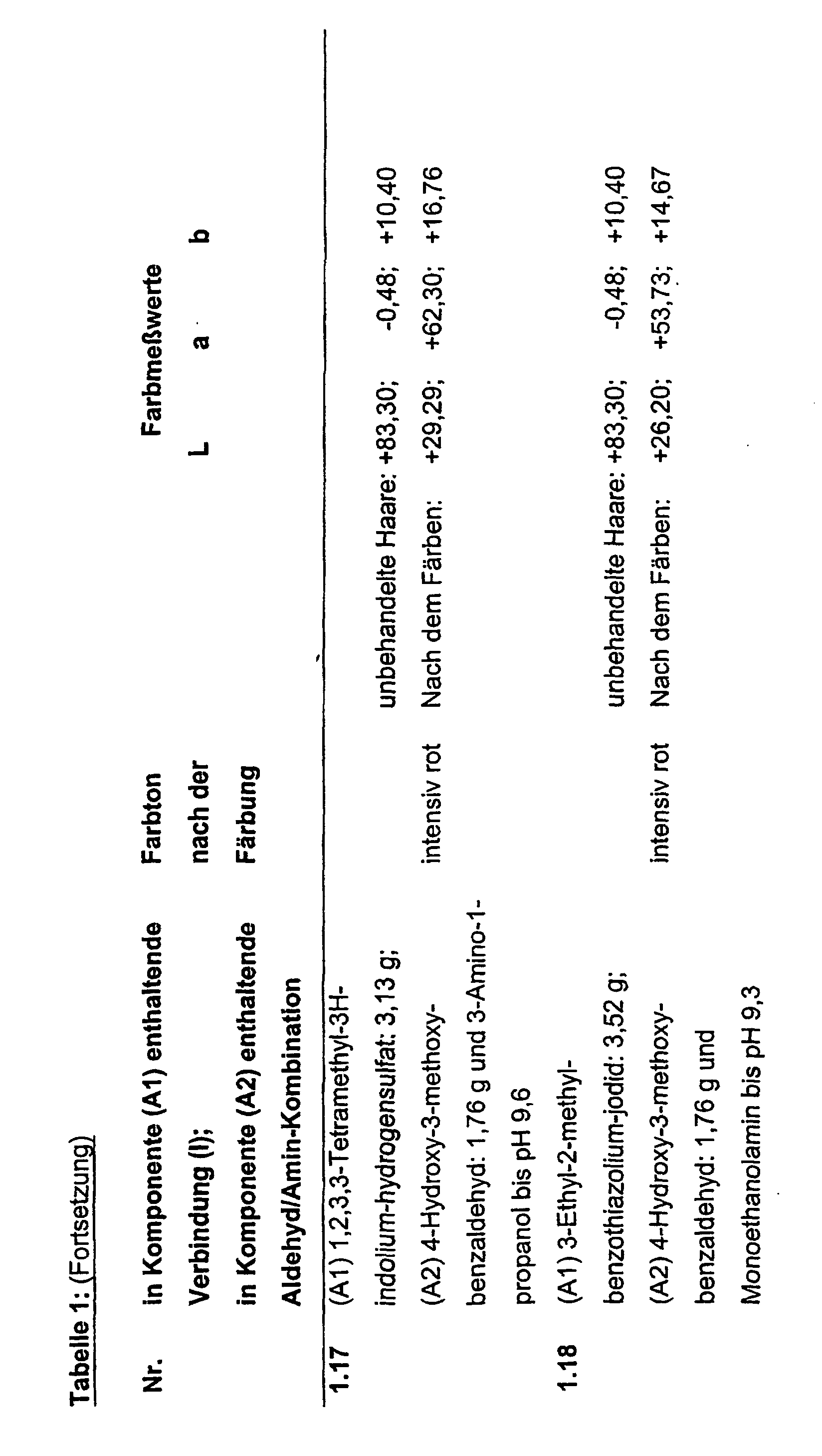

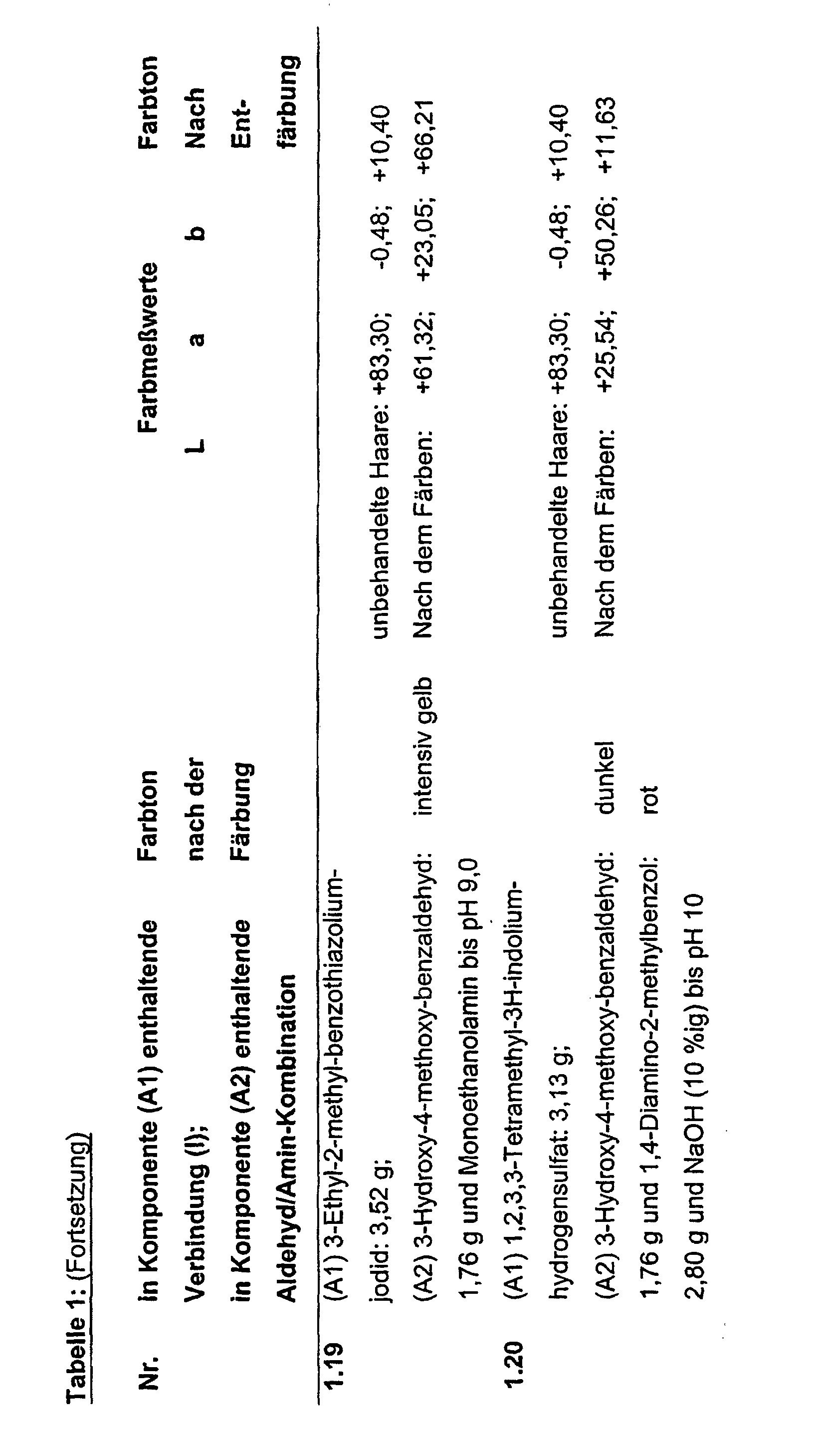

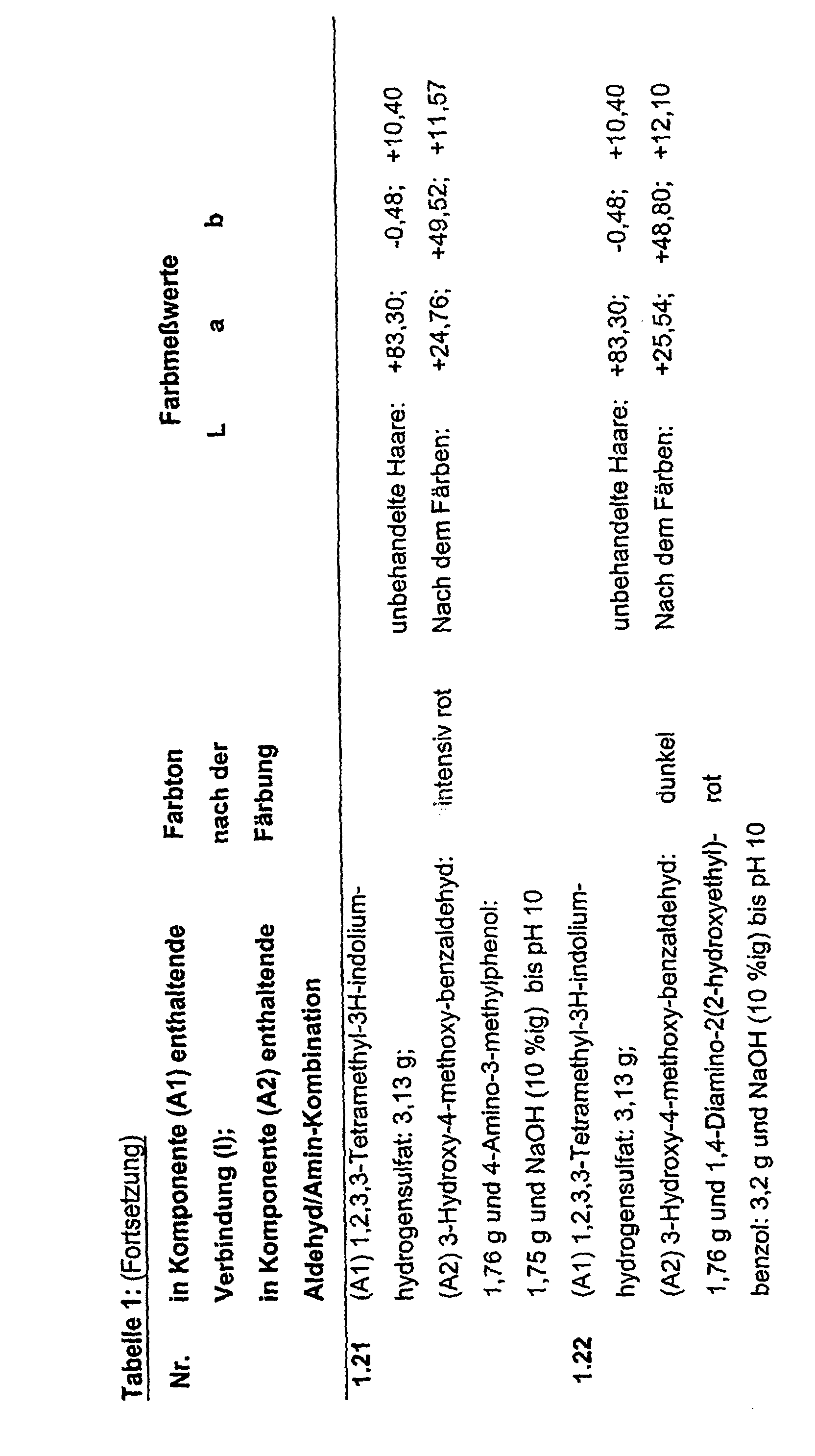

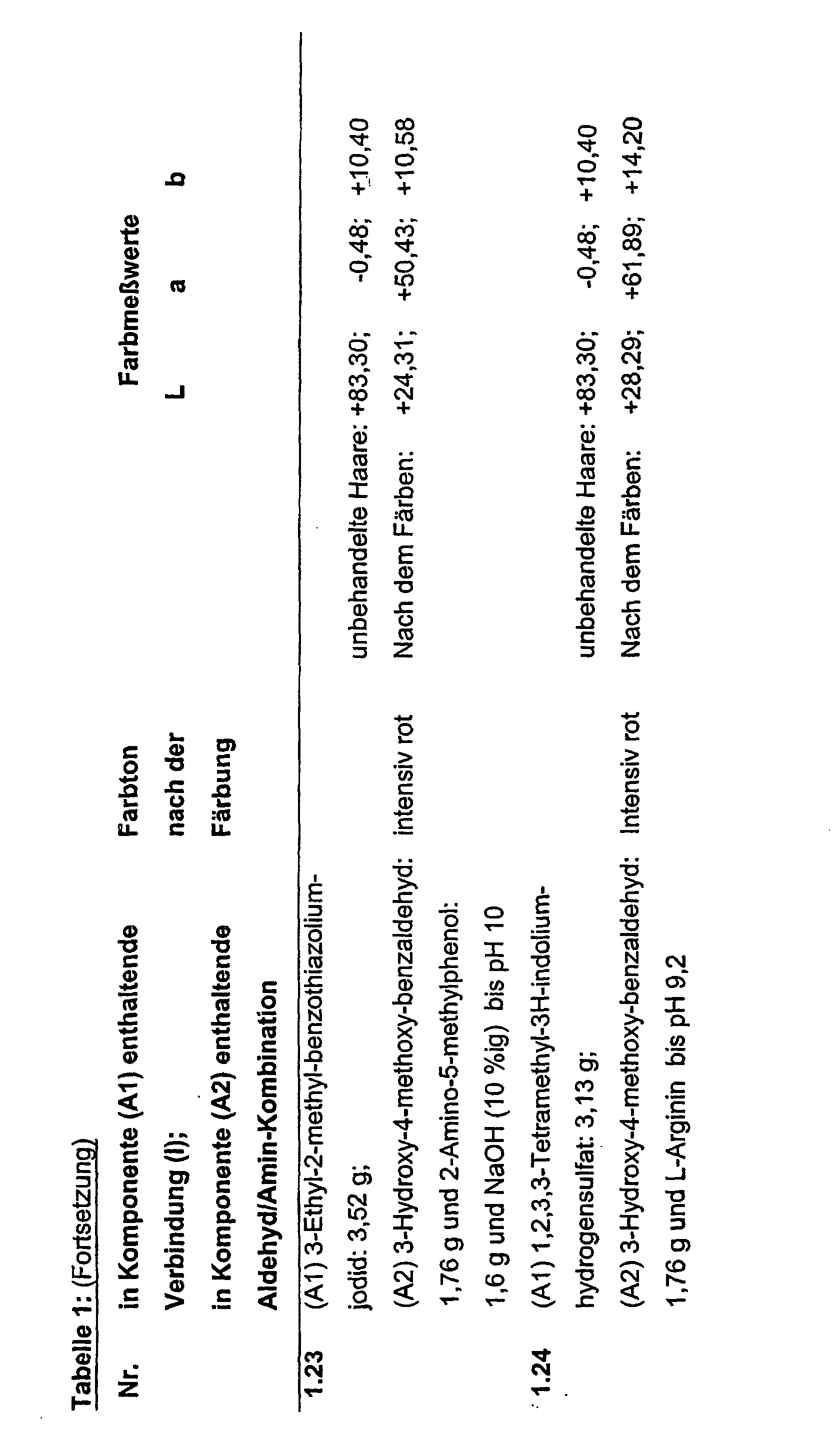

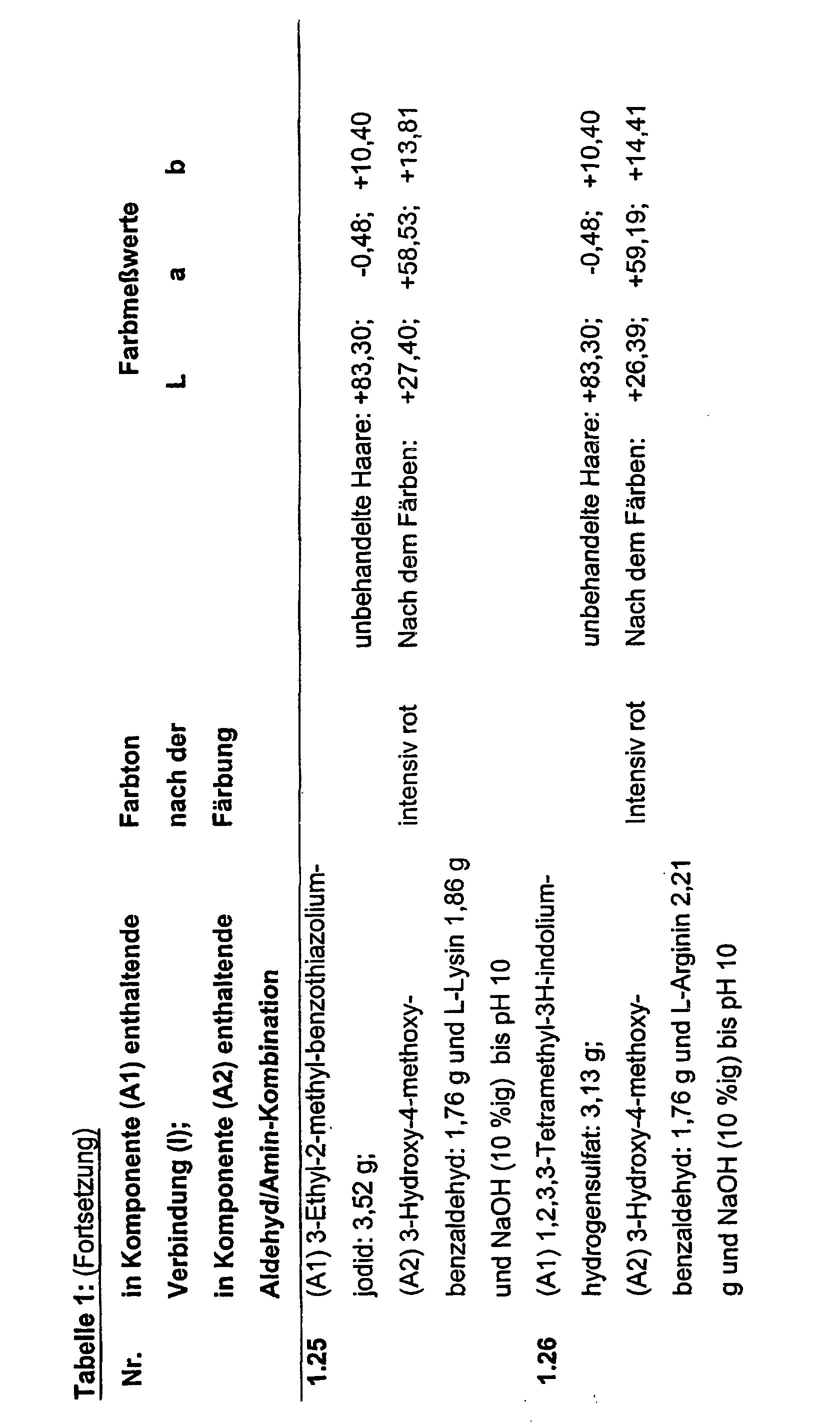

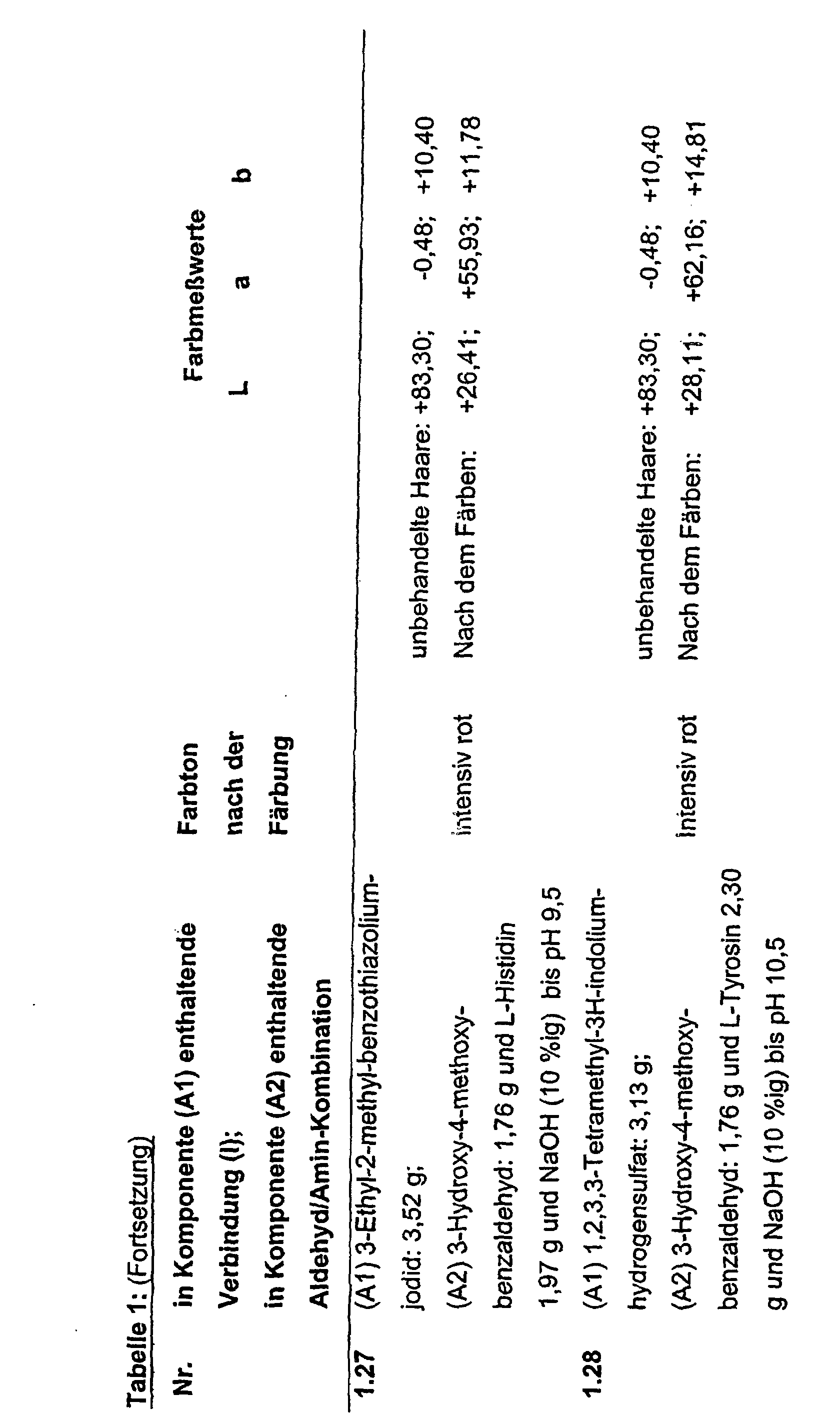

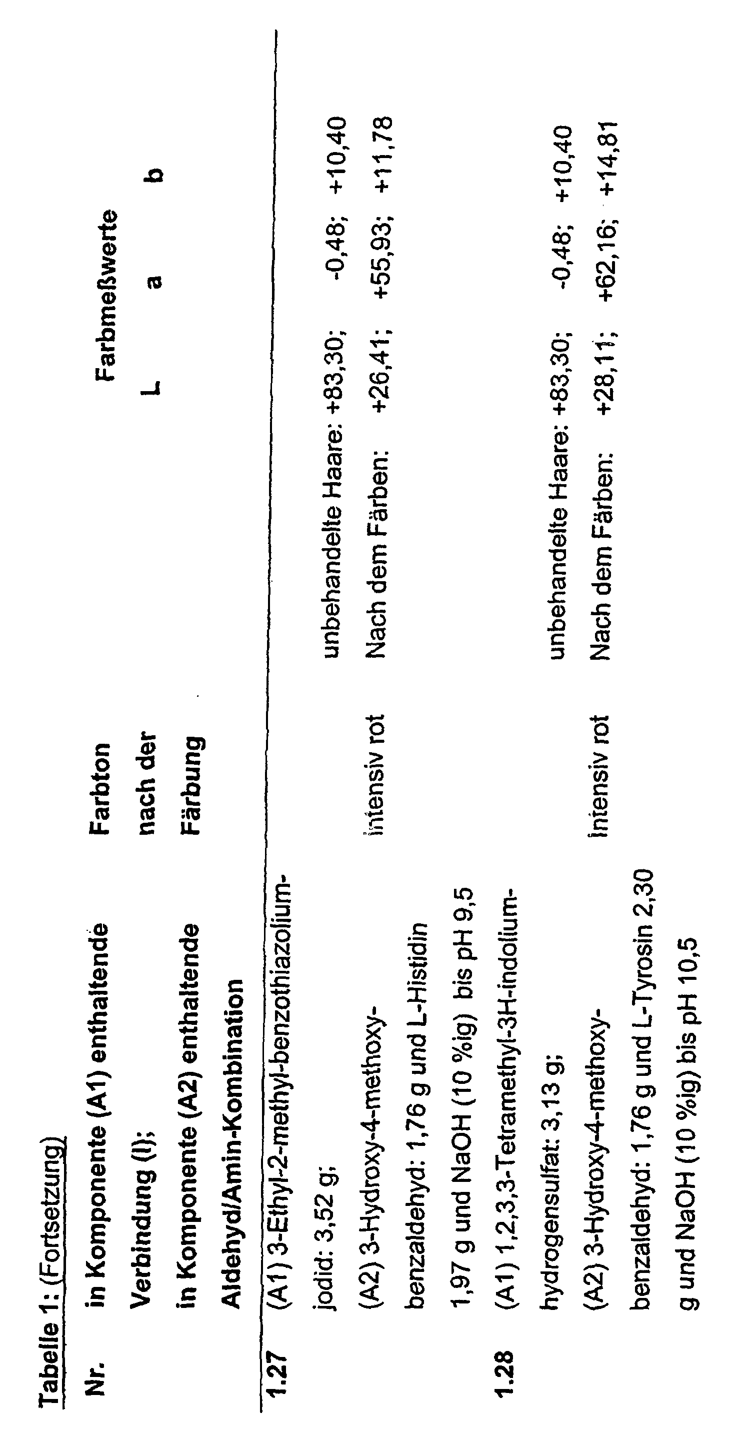

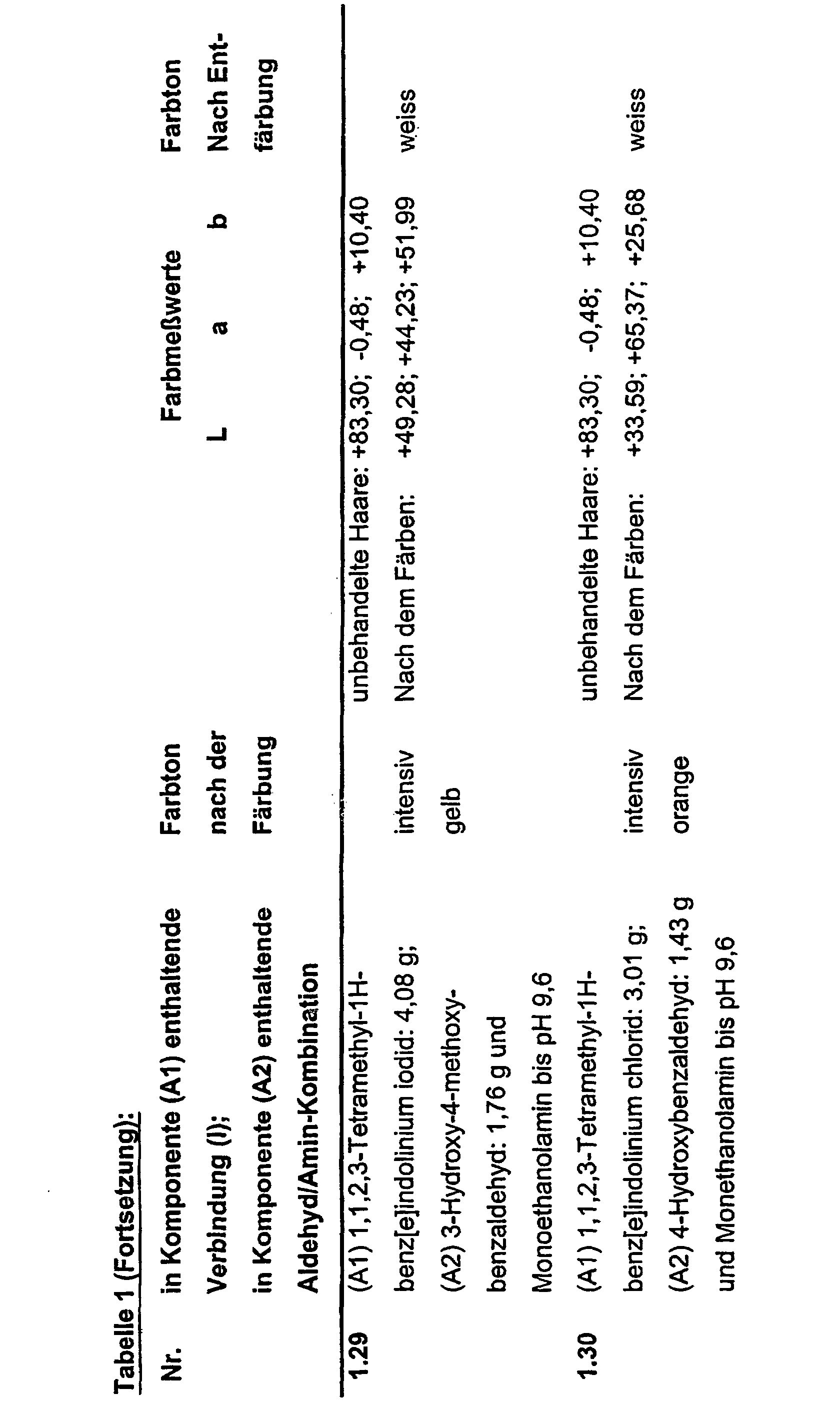

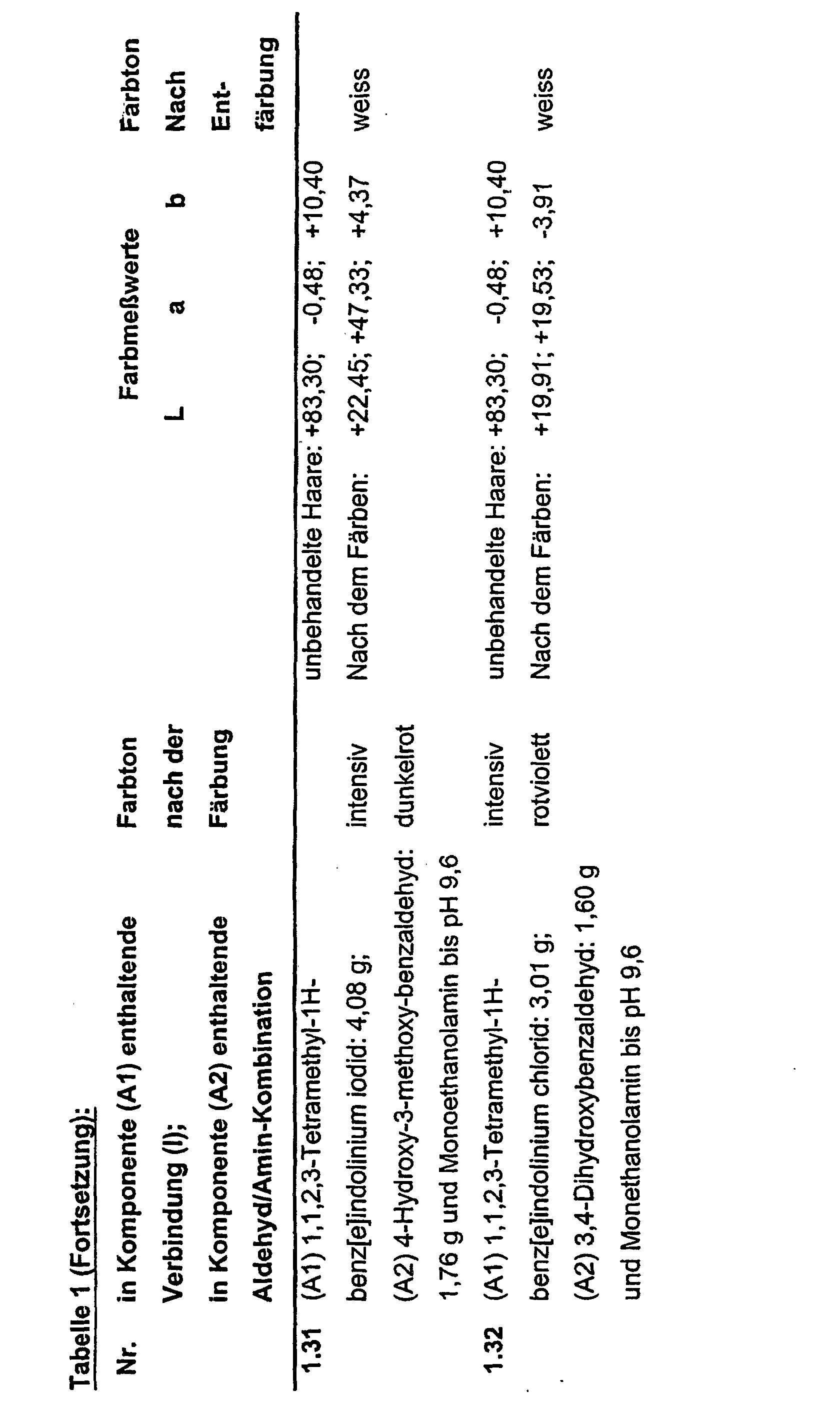

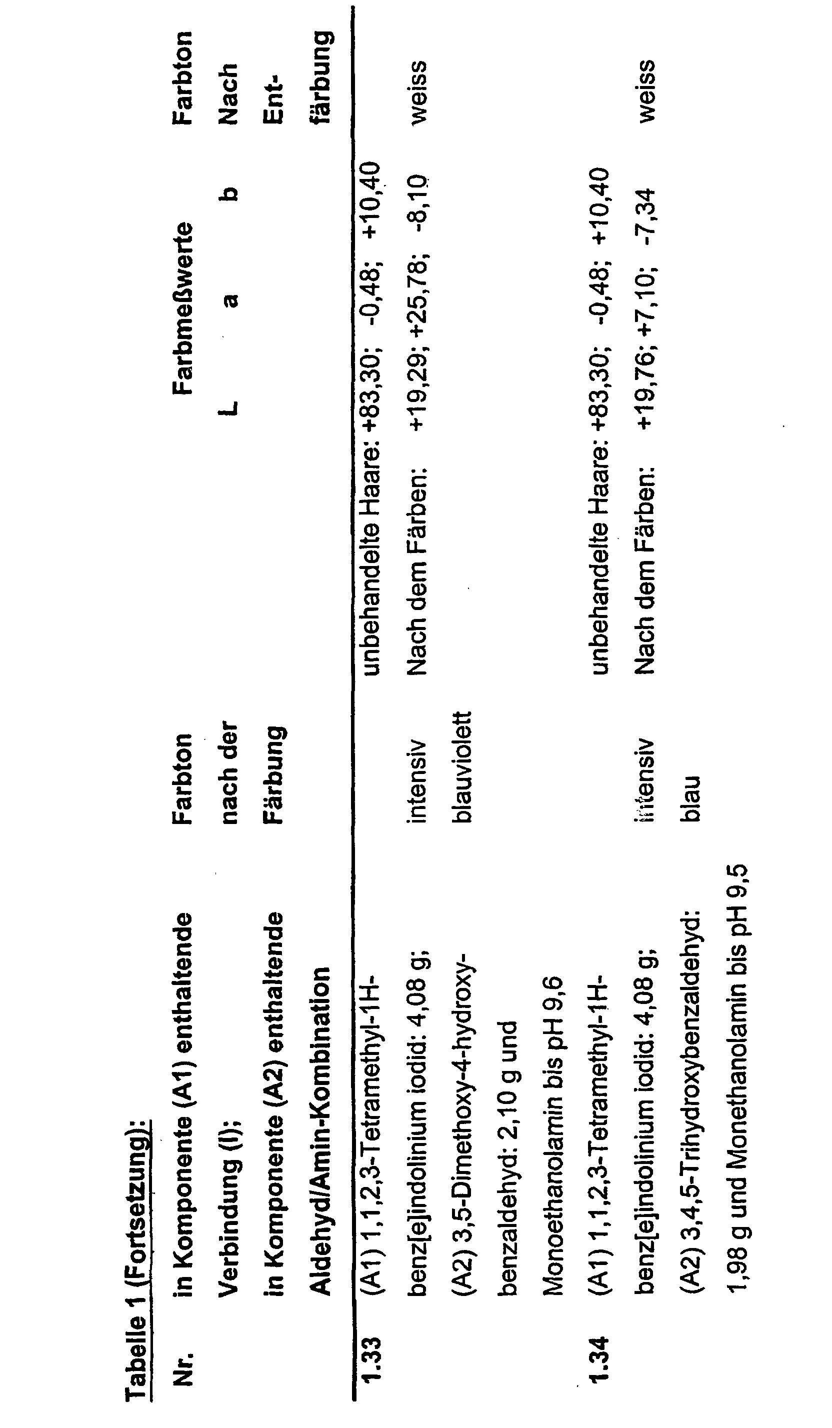

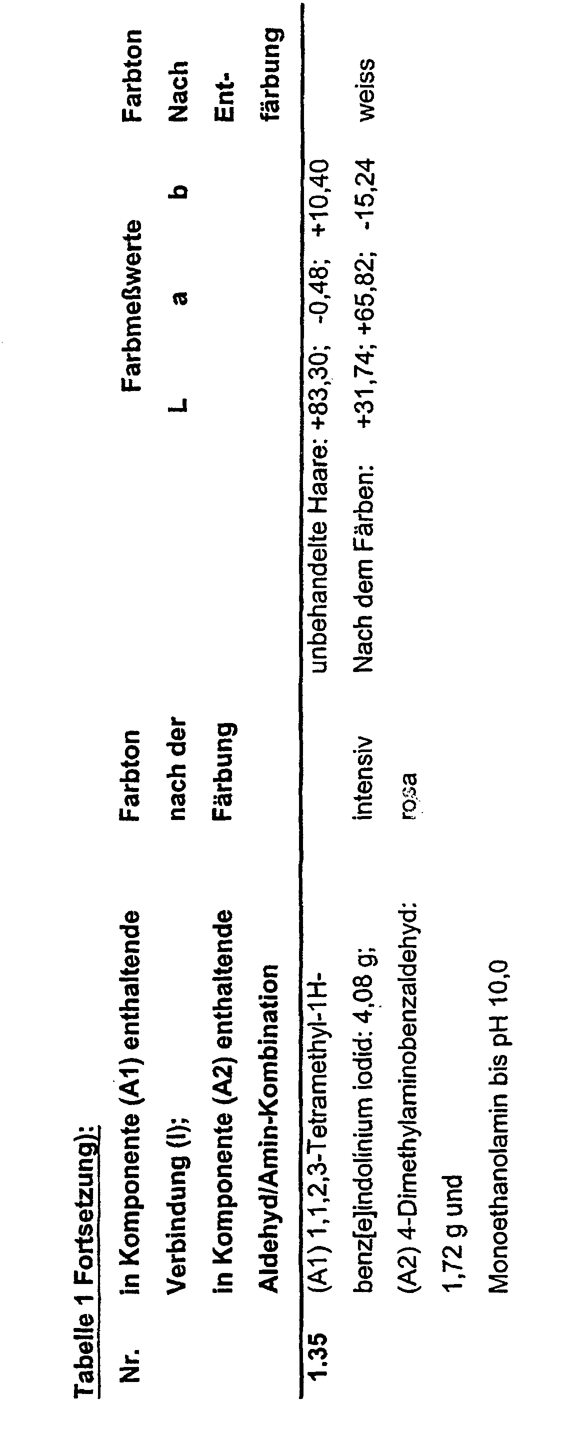

## Patentansprüche

1. Mittel zur Färbung von Fasern (A), insbesondere menschlichen Haaren, welches durch Vermischen einer ein Enamin enthaltenden Komponente (A1) und einer eine Carbonylverbindung und ein primäres Amin enthaltenden Komponente (A2) hergestellt wird und **dadurch gekennzeichnet ist, dass** die Komponente (A1) einen sauren pH-Wert aufweist und mindestens ein Enamin der Formel (I) oder dessen Säureadditionssalz der Formel (Ia) enthält, 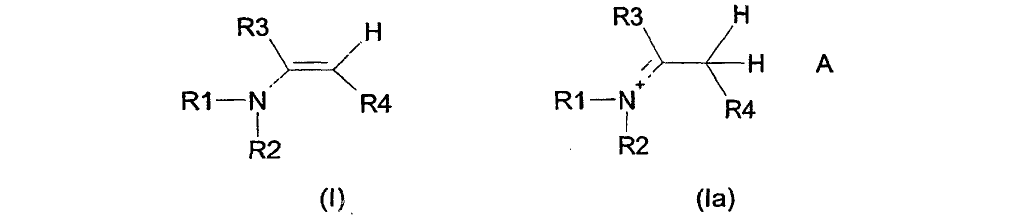 wobei gilt: **R1** gleich einem ein- oder mehrkernigen aromatischen Rest, insbesondere einem unsubstituierten 5- gliedrigen oder 6-gliedrigen Arylrest, einem unsubstituierten 5-gliedrigen oder 6-gliedrigen Heterozyklus, einem mit einer C1- bis C4-Alkylgruppe, einer C1- bis C4-Hydroxyalkylgruppe, einer Hydroxygruppe, einer Methoxygruppe, einer Dialkylaminogruppe oder einer Halogengruppe substituierten 5-gliedrigen oder 6-gliedrigen Arylrest, oder einem mit einer C1- bis C4-Alkylgruppe, einer C1-bis C4-Hydroxyalkylgruppe, einer Hydroxygruppe, einer Methoxygruppe, einer Dialkylaminogruppe oder einer Halogengruppe substituierten 5- gliedrigen oder 6-gliedrigen Heterozyklus;
**R2** gleich einer geradkettigen oder verzweigten C1- bis C8-Alkylgruppe, einer geradkettigen oder verzweigten C1- bis C8-Hydroxyalkylgruppe oder einer C1- bis C8-Alkoxyalkylgruppe, wobei jeweils zwischen den C-Atomen der Alkylkette Sauerstoffatome sitzen können, und
**R3** gleich einer geradkettigen oder verzweigten C1- bis C8-Alkylgruppe, einer C1- bis C8-Alkoxyalkylgruppe, einer geradkettigen oder verzweigten C1- bis C8-Alkylengruppe, einer C1- bis C8-Alkoxyalkylengruppe, einem Sauerstoffatom, einem Schwefelatom, einer -NH-Gruppe oder einer - NR-Gruppe, mit R gleich einer Alkylgruppe, Alkoxyalkylgruppe, Hydroxyalkylgruppe oder Wasserstoff; wobei die Reste **R1** und **R3** gemeinsam mit dem Stickstoffatom und dem Kohlenstoffatom der Enamingrundstruktur eine zyklische Verbindung bilden können; und **R4** gleich Wasserstoff, einer geradkettigen C1- bis C4-Alkylgruppe oder einer verzweigten C1- bis C4-Alkylgruppe; und **A**^{**-**} gleich dem Anion einer organischen oder anorganischen Säure ist; und die Komponente (A2) einen alkalischen pH-Wert aufweist und mindestens eine Carbonylverbindung und mindestens ein primäres Amin enthält.

2. Mittel nach Anspruch 1, **dadurch gekennzeichnet, dass** die Verbindung der Formel (I)/(Ia) ausgewählt ist aus Enaminen der Formeln (II) bis (IX), in denen **X** gleich einem mit zwei C1- bis C4-Alkylgruppen, welche gleich oder verschieden sein können, substituierten C-Atom, einem mit einer C1- bis C4-Alkylgruppe und einer Hydroxygruppe substituierten C-Atom, einem Schwefelatom, einem alkylierten Stickstoffatom, einem nichtalkylierten Stickstoffatom oder einem Sauerstoffatom ist; und **R2** gleich einer geradkettigen oder verzweigten C1- bis C8-Alkylgruppe, einer geradkettigen oder verzweigten C1- bis C8-Hydroxyalkylgruppe, oder einer C1- bis C8-Alkoxyalkylgruppe ist, wobei jeweils zwischen den C-Atomen der Alkylkette Sauerstoffatome sitzen können; **R4** gleich Wasserstoff, einer geradkettigen C1- bis C4-Alkylgruppe oder einer verzweigten C1- bis C4-Alkylgruppe ist; **R5, R6, R7** und **R8** unabhängig voneinander gleich Wasserstoff, einer geradkettigen oder verzweigten C1- bis C4-Alkylgruppe, einer geradkettigen oder verzweigten C1- bis C4-Hydroxyalkylgruppe, einer Hydroxygruppe, einer Methoxygruppe, einer Aminogruppe, einer Monoalkylaminogruppe, einer Dialkylaminogruppe, einer Benzylgruppe oder einem Halogenatom sind; **R9** gleich Wasserstoff, einer geradkettigen C1- bis C4-Alkylgruppe oder einer verzweigten C1- bis C4-Alkylgruppe ist; und **A**^{**-**} gleich Chlorid, Bromid, Jodid, Sulfat, Hydrogensulfat, Toluolsulfonat, Benzolsulfonat, Monomethylsulfat, Hexafluorphosphat, Hexafluorantimonat, Tetrafluorborat, Tetraphenylborat, Formiat, Acetat oder Propionat ist.

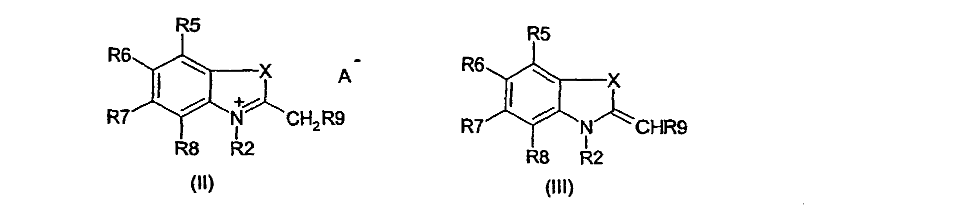

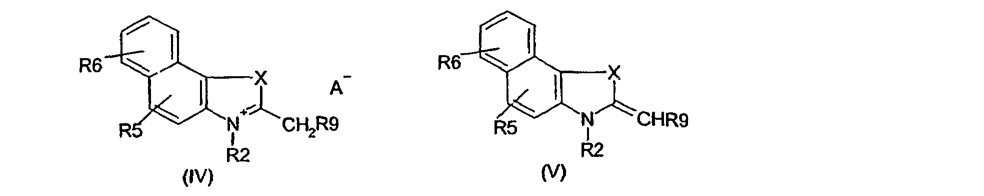

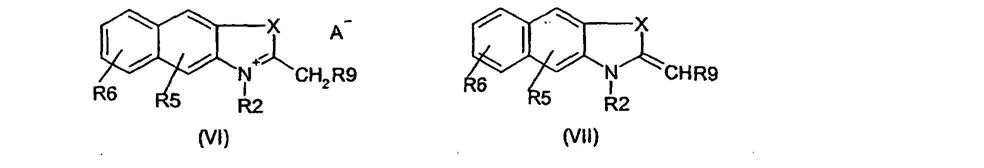

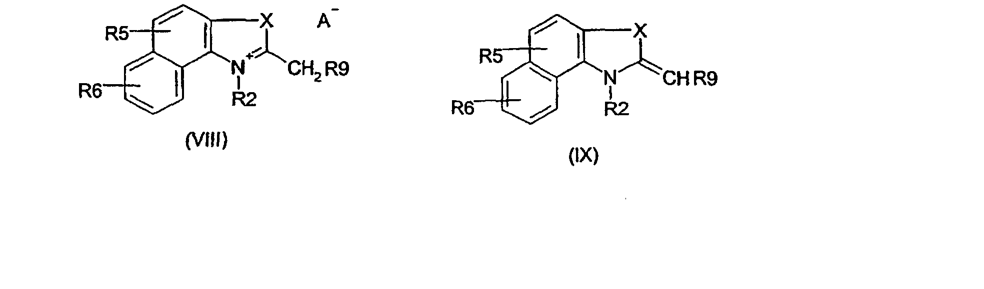

3. Mittel nach Anspruch 1 oder 2, **dadurch gekennzeichnet, dass** die Verbindung der Formeln (I) bis (IX) ausgewählt ist aus 1,3,3-Trimethyl-2-methylen-indolin sowie dessen Salzen, 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indolin sowie dessen Salzen, 5-Chloro-1,3,3-trimethyl-2-methylen-indolin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Amino-1,3,3-trimethyl-2-methylen-indolin sowie dessen Salzen, 6-Hydroxy-1,3,3-trimethyl-2-methylen-indolin sowie dessen Salzen, 6-Methoxy-1,3,3-trimethyl-2-methylen-indolin sowie dessen Salzen, 5-Methoxy-6-amino-1,3,3-trimethyl-2-methylen-indolin sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 4,5-Dihydroxy-1,3,3-trimethyl-2-methylen-indolin sowie dessen Salzen, 5,7-Dihydroxy-1,3,3-trimethyl-2-methylen-indolin sowie dessen Salzen, 5-Amino-6-methoxy-1,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 1-(2'-Hydroxyethyl)-3,3-dimethyl-2-methylen-indolin sowi dessen Salzen, 1,3,3-Trimethyl-2-methylen-3H-benz[e]indol sowie dessen Salzen und N-Ethyl-2-methylen-benzthiazol sowie dessen Salzen.

4. Mittel nach einem der Ansprüche 1 bis 3, **dadurch gekennzeichnet, dass** die Carbonylverbindung ausgewählt ist aus 4-Hydroxy-3-methoxy-benzaldehyd, 3-Hydroxy-4-methoxy-benzaldehyd, 3,4-Dihydroxybenzaldehyd, 4-Hydroxybenzaldehyd, 3,5-Dimethoxy-4-hydroxybenzaldehyd, 4-Dimethylaminobenzaldehyd, 4-Methyl-5-imidazolcarboxaldehyd, 4- Dimethylamino-zimtaldehyd, 4-Hydroxy-2-methoxy-benzaldehyd, 3,5-Dimethyl-4-hydroxybenzaldehyd, 4-Dimethylamino-2-methoxy-benzaldehyd, 2-Hydroxybenzaldehyd, 4-Hydroxy-1-naphthaldehyd, 4-Methoxy-1-naphthaldehyd, 4-Dimethylamino-1-naphthaldehyd, 4'-Hydroxy-biphenyl-1-carbaldehyd, 2-Hydroxy-3-methoxybenzaldehyd, 2,4-Dihydroxybenzaldehyd, 3,4-Dihydroxy-benzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxy-benzaldehyd, 2,5-Dimethoxy-benzaldehyd, 3,5-Dimethoxybenzaldehyd, 3,4-Dimethoxybenzaldehyd, Indol-3-carbaldehyd, Benzol-1,4-dicarbaldehyd, 4-Ethoxybenzaldehyd, 2-Methyl-1,4-naphthochinon, 4-Carboxybenzaldehyd, 4-Hydroxy-3-methoxyzimtaldehyd, 3,5-Dimethoxy-4-hydroxy-zimtaldehyd, 3-Methoxy-4-(1-pyrrolidinyl)-benzaldehyd, 4-Diethylamino-3-methoxybenzaldehyd, 1,2-Phthaldialdehyd, Pyrrol-2-aldehyd, Thiophen-2-aldehyd, Thiophen-3-aldehyd, Chromone-3-carboxaldehyd, 6-Methyl-4-oxo-1 (4H)-benzopyran-3-carbaldehyd, N-Methylpyrrol-2-aldehyd, 5-Methylfurfural, 6-Hydroxychromen-3-carboxaldehyd, 6-Methylindol-3-carboxaldehyd, 4-Dibutylaminobenzaldehyd, N-Ethylcarbazol-3-aldehyd, 4-Diethylamino-2-hydroxybenzaldehyd, 3,4-Dimethoxy-5-hydroxybenzaldehyd, 5-(4-(Diethylamino)-phenyl)-2,4-pentadienal, 2,3-Thiophendicarbox-aldehyd, 2,5-Thiophendicarboxaldehyd, 2-Methoxy-1-naphthaldehyd, 3-Ethoxy-4-hydroxybenzaldehyd, 2-Nitrobenzaldehyd, 3-Nitro-benzaldehyd und 4-Nitrobenzaldehyd.

5. Mittel nach einem der Ansprüche 1 bis 4, **dadurch gekennzeichnet, dass** das primäre Amin ausgewählt ist aus Alkanolaminen, aromatischen Aminen und Aminosäuren.

6. Mittel nach Anspruch 5, **dadurch gekennzeichnet, dass** das primäre Amin ausgewählt ist aus Monoethanolamin, Isopropanolamin, 1-Amino-2-propanol, 3-Amino-1-propanol und Arginin.

7. Mittel nach einem der Ansprüche 1 bis 6, **dadurch gekennzeichnet, dass** die Komponente (A1) einen pH-Wert von 1 bis 4,5 hat.

8. Mittel nach einem der Ansprüche 1 bis 7, **dadurch gekennzeichnet, dass** die Komponente (A2) einen pH-Wert von 7,5 bis 12 hat.

9. Mittel nach einem der Ansprüche 1 bis 8, **dadurch gekennzeichnet, dass** es die Verbindungen der Formel (I) bis (IX), die Carbonylverbindungen und die Amine, bezogen auf die gebrauchsfertige Zubereitung, jeweils in einer Gesamtmenge von 0,01 bis 10 Gewichtsprozent enthält.

10. Verfahren zur temporären Färbung von Fasern, insbesondere Haaren, bei dem die Faser mit einem Mittel nach einem der Ansprüche 1 bis 9 gefärbt wird und zu einem beliebigen späteren Zeitpunkt wieder entfärbt wird, **dadurch gekennzeichnet, dass** man zur Entfärbung eine sulfithaltige Zubereitung für eine Dauer von 5 bis 60 Minuten bei einer Temperatur von 20 bis 50 °C auf die gefärbte Faser einwirken läßt.

11. Mehrkomponenten-Kit, bestehend aus einer ein Enamin der Formel (I) enthaltenden Komponente (A1) und einer eine Carbonylverbindung und ein primäres Amin enthaltenden Komponente (A2).

## Claims

1. Agent for colouring fibres (A), in particular human hair, which is prepared by mixing a component (A1) comprising an enamine, and a component (A2) comprising a carbonyl compound and a primary amine and **characterized in that** the component (A1) has an acidic pH and comprises at least one enamine of the formula (I) or its acid addition salt of the formula (Ia), 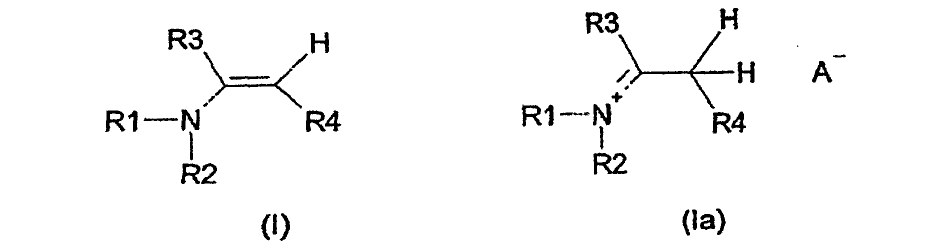 where: **R1** is a mono- or polynuclear aromatic radical, in particular an unsubstituted 5-membered or 6-membered aryl radical, an unsubstituted 5-membered or 6-membered heterocycle, a 5-membered or 6-membered aryl radical substituted by a C1-to C4-alkyl group, a C1- to C4-hydroxyalkyl group, a hydroxyl group, a methoxy group, a dialkylamino group or a halogen group, or a 5-membered or 6-membered heterocycle substituted by a C1- to C4-alkyl group, a C1- to C4-hydroxyalkyl group, a hydroxyl group, a methoxy group, a dialkylamino group or a halogen group;
**R2** is a straight-chain or branched C1- to C8-alkyl group, a straight-chain or branched C1- to C8-hydroxylalkyl group or a C1- to C8-alkoxyalkyl group, where in each case oxygen atoms may sit between the carbon atoms of the alkyl chain,
**R3** is a straight-chain or branched C1- to C8-alkyl group, a C1- to C8-alkoxyalkyl group, a straight-chain or branched C1- to C8-alkylene group, a C1- to C8-alkoxyalkylene group, an oxygen atom, a sulphur atom, a -NH group or a -NR group, where **R** is an alkyl group, alkoxyalkyl group, hydroxyalkyl group or hydrogen; where the radicals **R1** and **R3**, together with the nitrogen atom and the carbon atom of the enamine base structure, can form a cyclic compound; and **R4** is hydrogen, a straight-chain C1- to C4-alkyl group or a branched C1- to C4-alkyl group; and **A**^{**-**} is the anion of an organic or inorganic acid; and component (A2) has an alkaline pH and comprises at least one carbonyl compound and at least one primary amine.

2. Agent according to Claim 1, **characterized in that** the compound of the formula (I)/(Ia) is chosen from enamines of the formulae (II) to (IX), in which **X** is a carbon atom substituted by two C1- to C4-alkyl groups, which may be identical or different, a carbon atom substituted by one C1- to C4-alkyl group and one hydroxyl group, a sulphur atom, an alkylated nitrogen atom, a nonalkylated nitrogen atom or an oxygen atom; and **R2** is a straight-chain or branched C1- to C8-alkyl group, a straight-chain or branched C1- to C8-hydroxy-alkyl group, or a C1- to C8-alkoxyalkyl group, where in each case oxygen atoms may sit between the carbon atoms of the alkyl chain; **R4** is hydrogen, a straight-chain C1- to C4-alkyl group or a branched C1- to C4-alkyl group; **R5, R6, R7** and **R8**, independently of one another, are hydrogen, a straight-chain or branched C1- to C4-alkyl group, a straight-chain or branched C1- to C4-hydroxyalkyl group, a hydroxyl group, a methoxy group, an amino group, a monoalkylamino group, a dialkylamino group, a benzyl group or a halogen atom;
**R9** is hydrogen, a straight-chain C1- to C4-alkyl group or a branched C1- to C4-alkyl group; and **A**^{**-**} is chloride, bromide, iodide, sulphate, hydrogensulphate, toluenesulphonate, benzenesulphonate, monomethyl sulphate, hexafluorophosphate, hexafluoroantimonate, tetrafluoroborate, tetraphenylborate, formate, acetate or propionate.

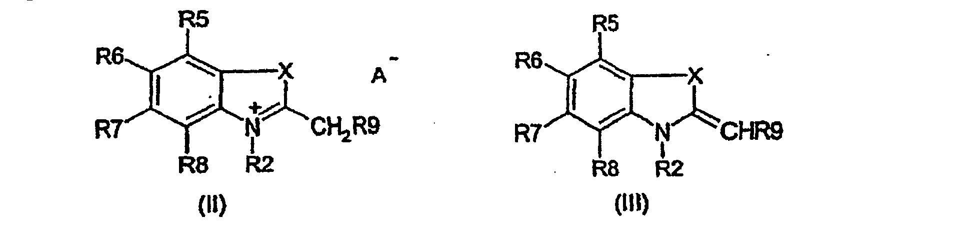

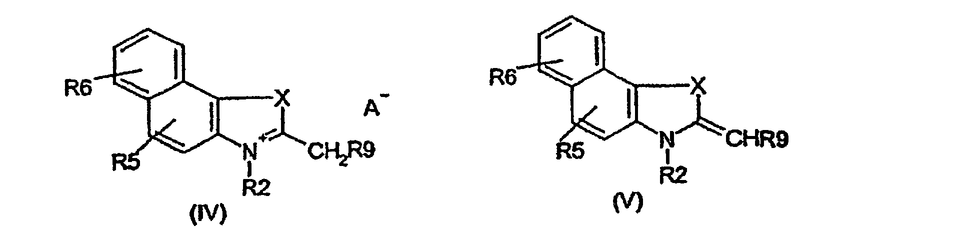

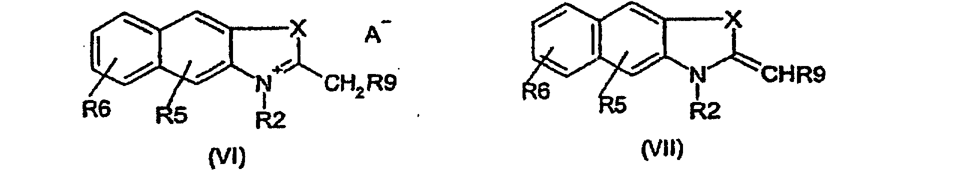

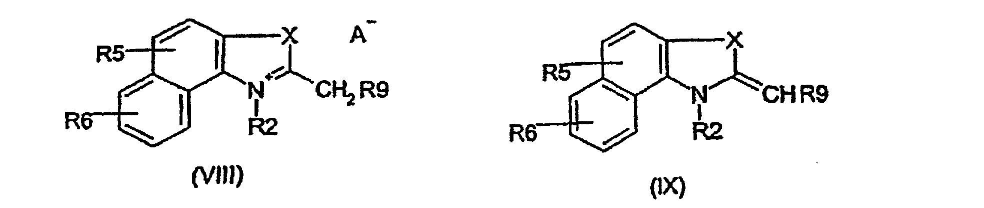

3. Agent according to Claim 1 or 2, **characterized in that** the compound of the formulae (I) to (IX) is chosen from 1,3,3-trimethyl-2-methyleneindoline and salts thereof, 1,3,3,4-tetramethyl-2-methyleneindoline and salts thereof, 1,3,3,5-tetramethyl-2-methyleneindoline and salts thereof, 1,3,3,6-tetramethyl-2-methyleneindoline and salts thereof, 1,3,3,7-tetramethyl-2-methyleneindoline and salts thereof, 1,3,3,6,7-pentamethyl-2-methyleneindoline and salts thereof, 1,3,3,5,7-pentamethyl-2-methyleneindoline and salts thereof, 1,3,3,4,7-pentamethyl-2-methyleneindoline and salts thereof, 5-chloro-1,3,3,-trimethyl-2-methyleneindoline and salts thereof, 5-fluoro-1,3,3,-trimethyl-2-methyleneindoline and salts thereof, 5-isopropyl-1,3,3,-trimethyl-2-methyleneindoline and salts thereof, 5-hydroxy-1,3,3,-trimethyl-2-methyleneindoline and salts thereof, 5-methoxy-1,3,3,-trimethyl-2-methyleneindoline and salts thereof, 5-amino-1,3,3,-trimethyl-2-methyleneindoline and salts thereof, 6-hydroxy-1,3,3,-trimethyl-2-methyleneindoline and salts thereof, 6-methoxy-1,3,3,-trimethyl-2-methyleneindoline and salts thereof, 5-methoxy-6-amino-1,3,3,-trimethyl-2-methyleneindoline and salts thereof, 5,6-dihydroxy-1,3,3,-trimethyl-2-methyleneindoline and salts thereof, 5,6-dimethoxy-1,3,3,-trimethyl-2-methyleneindoline and salts thereof, 4,5-dihydroxy-1,3,3,-trimethyl-2-methyleneindoline and salts thereof, 5,7-dihydroxy-1,3,3,-trimethyl-2-methyleneindoline and salts thereof, 5-amino-6-methoxy-1,3,3,-trimethyl-2-methyleneindoline and salts thereof, 5-amino-7-hydroxy-1,3,3,-trimethyl-2-methyleneindoline and salts thereof, 5-hydroxy-7-amino-1,3,3,-trimethyl-2-methyleneindoline and salts thereof, 1-(2'-hydroxyethyl)-3,3-dimethyl-2-methyleneindoline and salts thereof, 1,3,3-trimethyl-2-methylene-3H-benz[e]indole and salts thereof and N-ethyl-2-methylenebenzothiazole and salts thereof.

4. Agent according to one of Claims 1 to 3, **characterized in that** the carbonyl compound is chosen from 4-hydroxy-3-methoxybenzaldehyde, 3-hydroxy-4-methoxybenzaldehyde, 3,4-dihydroxybenzaldehyde, 4-hydroxybenzaldehyde, 3,5-dimethoxy-4-hydroxybenzaldehyde, 4-dimethylaminobenzaldehyde, 4-methyl-5-imidazolecarboxaldehyde, 4-dimethylaminocinnamaldehyde, 4-hydroxy-2-methoxybenzaldehyde, 3,5-dimethyl-4-hydroxybenzaldehyde, 4-dimethylamino-2-methoxybenzaldehyde, 2-hydroxybenzaldehyde, 4-hydroxy-1-naphthaldehyde, 4-methoxy-1-naphthaldehyde, 4-dimethylamino-1-naphthaldehyde, 4'-hydroxybiphenyl-1-carbaldehyde, 2-hydroxy-3-methoxybenzaldehyde, 2,4-dihydroxybenzaldehyde, 3,4-dihydroxybenzaldehyde, 2,5-dihydroxybenzaldehyde, 2,3,4-trihydroxybenzaldehyde, 3,4,5-trihydroxybenzaldehyde, 2,4,6-trihydroxybenzaldehyde, 2,4-dimethoxybenzaldehyde, 2,3-dimethoxybenzaldehyde, 2,5-dimethoxybenzaldehyde, 3,5-dimethoxybenzaldehyde, 3,4-dimethoxybenzaldehyde, indole-3-carbaldehyde, benzene-1,4-dicarbaldehyde, 4-ethoxybenzaldehyde, 2-methyl-1,4-naphthoquinone, 4-carboxybenzaldehyde, 4-hydroxy-3-methoxycinnamaldehyde, 3,5-dimethoxy-4-hydroxycinnamaldehyde, 3-methoxy-4-(1-pyrrolidinyl)benzaldehyde, 4-diethylamino-3-methoxybenzaldehyde, 1,2-phthaldialdehyde, pyrrole-2-aldehyde, thiophene-2-aldehyde, thiophene-3-aldehyde, chromone-3-carboxaldehyde, 6-methyl-4-oxo-1(4H)-benzopyran-3-carbaldehyde, N-methylpyrrole-2-aldehyde, 5-methylfurfural, 6-hydroxychromene-3-carboxaldehyde, 6-methylindole-3-carboxaldehyde, 4-dibutylaminobenzaldehyde, N-ethylcarbazole-3-aldehyde, 4-diethylamino-2-hydroxybenzaldehyde, 3,4-dimethoxy-5-hydroxybenzaldehyde, 5-(4-(diethylamino)phenyl)-2,4-pentadienal, 2,3-thiophenedicarboxaldehyde, 2,5-thiophenedicarboxaldehyde, 2-methoxy-1-naphthaldehyde, 3-ethoxy-4-hydroxybenzaldehyde, 2-nitrobenzaldehyde, 3-nitrobenzaldehyde and 4-nitrobenzaldehyde.

5. Agent according to one of Claims 1 to 4, **characterized in that** the primary amine is chosen from alkanolamines, aromatic amines and amino acids.

6. Agent according to Claim 5, **characterized in that** the primary amine is chosen from monoethanolamine, isopropanolamine, 1-amino-2-propanol, 3-amino-1-propanol and arginine.

7. Agent according to one of Claims 1 to 6, **characterized in that** component (A1) has a pH of from 1 to 4.5.

8. Agent according to one of Claims 1 to 7, **characterized in that** component (A2) has a pH of from 7.5 to 12.

9. Agent according to one of Claims 1 to 8, **characterized in that** it comprises compounds of the formula (I) to (IX), the carbonyl compounds and the amines, based on the ready-to-use preparation, in each case in a total amount of from 0.01 to 10 percent by weight.

10. Method of temporarily colouring fibres, in particular hair, in which the fibre is coloured with an agent according to one of Claims 1 to 9 and decoloured again at a later time as desired, **characterized in that**, for the decolouring, a sulphite-containing preparation is left to act on the coloured fibre for a period of from 5 to 60 minutes at a temperature of from 20 to 50°C.

11. Multicomponent kit consisting of a component (A1) comprising an enamine of the formula (I), and a component (A2) comprising a carbonyl compound and a primary amine.

## Revendications

1. Composition pour la teinture de fibres (A), en particulier de cheveux humains, qui est préparée par mélange d'un composant (A1) contenant une énamine et d'un composant (A2) contenant un composé carbonyle et une amine primaire, et **caractérisée en ce que** le composant (A1) présente un pH acide et contient au moins une énamine de formule (I) ou un sel d'addition avec un acide de celle-ci, de formule (Ia), 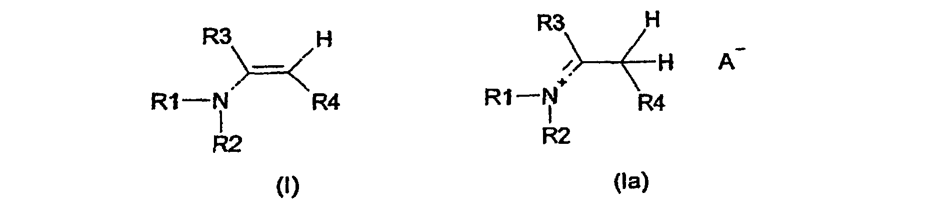 formules dans lesquelles : **R1** représente un radical aromatique mono- ou polynucléaire, en particulier un radical aryle à 5 chaînons ou 6 chaînons, non substitué, un hétérocycle à 5 chaînons ou 6 chaînons, non substitué, un radical aryle à 5 chaînons ou 6 chaînons, substitué par un groupe alkyle en C₁-C₄, un groupe hydroxyalkyle en C₁-C₄, un groupe hydroxy, un groupe méthoxy, un groupe dialkylamino ou un atome d'halogène, ou représente un hétérocycle à 5 chaînons ou 6 chaînons, substitué par un groupe alkyle en C₁-C₄, un groupe hydroxyalkyle en C₁-C₄, un groupe hydroxy, un groupe méthoxy, un groupe dialkylamino ou un atome d'halogène ;
**R2** représente un groupe alkyle en C₁-C₈ à chaîne droite ou ramifié, un groupe hydroxyalkyle en C₁-C₈ à chaîne droite ou ramifié ou un groupe alcoxy(C₁-C₈)alkyle, des atomes d'oxygène pouvant dans chaque cas se trouver entre les atomes de carbone de la chaîne alkyle, et
**R3** représente un groupe alkyle en C₁-C₈ à chaîne droite ou ramifié, un groupe alcoxy(C₁-C₈)alkyle, un groupe alkylène en C₁-C₈ à chaîne droite ou ramifié, un groupe alcoxy(C₁-C₈)alkylène, un atome d'oxygène, un atome de soufre, un groupe -NH ou un groupe -NR, R représentant un groupe alkyle, un groupe alcoxyalkyle, un groupe hydroxyalkyle ou un atome d'hydrogène ; les radicaux **R1** à **R3** pouvant former ensemble, avec l'atome d'azote et l'atome de carbone de la structure de base énamine, un composé cyclique ; et **R4** représente un atome d'hydrogène, un groupe alkyle en C₁-C₄ à chaîne droite ou un groupe alkyle en C₁-C₄ ramifié ; et **A** représente l'anion d'un acide organique ou minéral ; et le composant (A2) présente un pH alcalin et contient au moins un composé carbonyle et au moins une amine primaire.

2. Composition selon la revendication 1, **caractérisée en ce que** le composé de formule (I)/(Ia) est choisi parmi des énamines de formules (II) à (IX), dans lesquelles X représente un atome de carbone substitué par deux groupes alkyle en C₁-C₄ qui peuvent être identiques ou différents, un atome de carbone substitué par un groupe alkyle en C₁-C₄ et un groupe hydroxy, un atome de soufre, un atome d'azote alkylé, un atome d'azote non alkylé ou un atome d'oxygène ; et **R2** représente un groupe alkyle en C₁-C₈ à chaîne droite ou ramifié, un groupe hydroxyalkyle en C₁-C₈ à chaîne droite ou ramifié, ou un groupe alcoxy(C₁-C₈)alkyle, des atomes d'oxygène pouvant dans chaque cas se trouver entre les atomes de carbone de la chaîne alkyle ; **R4** représente un atome d'hydrogène, un groupe alkyle en C₁-C₄ à chaîne droite ou un groupe alkyle en C₁-C₄ ramifié ; **R5**, **R6**, **R7** et **R8** représentent, indépendamment les uns des autres, un atome d'hydrogène, un groupe alkyle en C₁-C₄ à chaîne droite ou ramifié, un groupe hydroxyalkyle en C₁-C₄ à chaîne droite ou ramifié, un groupe hydroxy, un groupe méthoxy, un groupe amino, un groupe monoalkylamino, un groupe dialkylamino, un groupe benzyle ou un atome d'halogène ; **R9** représente un atome d'hydrogène, un groupe alkyle en C₁-C₄ à chaîne droite ou un groupe alkyle en C₁-C₄ ramifié ; et **A**⁻ représente l'anion chlorure, bromure, iodure, sulfate, hydrogénosulfate, toluènesulfonate, benzènesulfonate, monométhylsulfate, hexafluorophosphate, hexafluoroantimoniate, tétrafluoroborate, tétraphénylborate, formiate, acétate ou propionate.

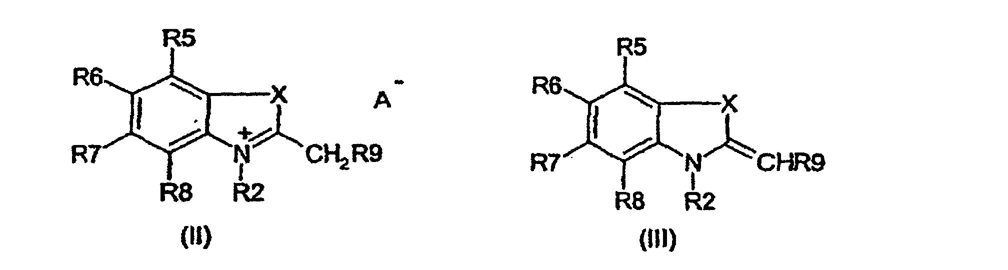

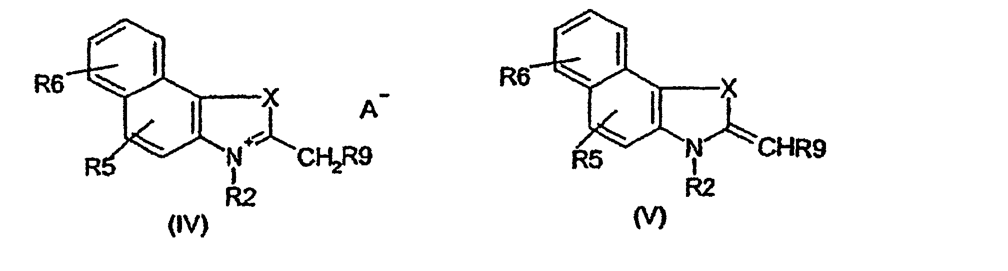

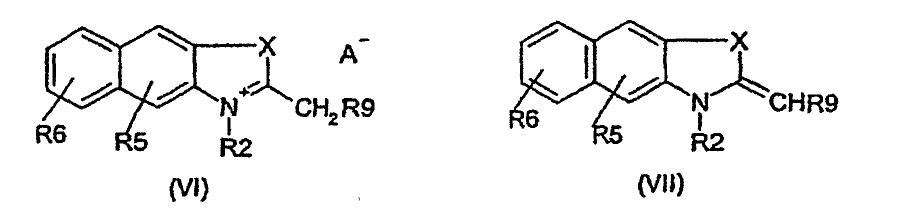

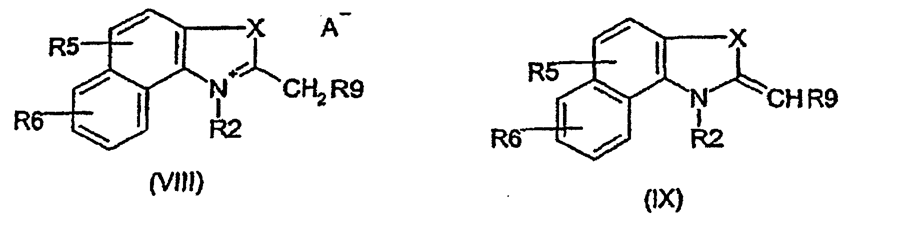

3. Composition selon la revendication 1 ou 2, **caractérisée en ce que** le composé de formules (I) à (IX) est choisi parmi la 1,3,3-triméthyl-2-méthylène-indoline ainsi que ses sels, la 1,3,3,4-tétraméthyl-2-méthylène-indoline ainsi que ses sels, la 1,3,3,5-tétraméthyl-2-méthylène-indoline ainsi que ses sels, la 1,3,3,6-tétraméthyl-2-méthylène-indoline ainsi que ses sels, la 1,3,3,7-tétraméthyl-2-méthylène-indoline ainsi que ses sels, la 1,3,3,6,7-pentaméthyl-2-méthylène-indoline ainsi que ses sels, la 1,3,3,5,7-pentaméthyl-2-méthylène-indoline ainsi que ses sels, la 1,3,3,4,7-pentaméthyl-2-méthylène-indoline ainsi que ses sels, la 5-chloro-1,3,3-triméthyl-2-méthylène-indoline ainsi que ses sels, la 5-fluoro-1,3,3-triméthyl-2-méthylène-indoline ainsi que ses sels, la 5-isopropyl-1,3,3-triméthyl-2-méthylèneindoline ainsi que ses sels, la 5-hydroxy-1,3,3-triméthyl-2-méthylène-indoline ainsi que ses sels, la 5-méthoxy-1,3,3-triméthyl-2-méthylène-indoline ainsi que ses sels, la 5-amino-1,3,3-triméthyl-2-méthylène-indoline ainsi que ses sels, la 6-hydroxy-1,3,3-triméthyl-2-méthylène-indoline ainsi que ses sels, la 6-méthoxy-1,3,3-triméthyl-2-méthylène-indoline ainsi que ses sels, la 5-méthoxy-6-amino-1,3,3-triméthyl-2-méthylène-indoline ainsi que ses sels, la 5,6-dihydroxy-1,3,3-triméthyl-2-méthylène-indoline ainsi que ses sels, la 5,6-diméthoxy-1,3,3-triméthyl-2-méthylène-indoline ainsi que ses sels, la 4,5-dihydroxy-1,3,3-triméthyl-2-méthylène-indoline ainsi que ses sels, la 5,7-dihydroxy-1,3,3-triméthyl-2-méthylène-indoline ainsi que ses sels, la 5-amino-6-méthoxy-1,3,3-triméthyl-2-méthylène-indoline ainsi que ses sels, la 5-amino-7-hydroxy-1,3,3-triméthyl-2-méthylène-indoline ainsi que ses sels, la 5-hydroxy-7-amino-1,3,3-triméthyl-2-méthylène-indoline ainsi que ses sels, la 1-(2'-hydroxyéthyl)-3,3-diméthyl-2-méthylène-indoline ainsi que ses sels, le 1,3,3-triméthyl-2-méthylène-3H-benz[e]indole ainsi que ses sels et le N-éthyl-2-méthylbenzothiazole ainsi que ses sels.

4. Composition selon l'une quelconque des revendications 1 à 3, **caractérisée en ce que** le composé carbonyle est choisi parmi le 4-hydroxy-3-méthoxybenzaldéhyde, le 3-hydroxy-4-méthoxybenzaldéhyde, le 3,4-dihydroxybenzaldéhyde, le 4-hydroxybenzaldéhyde, le 3,5-diméthoxy-4-hydroxybenzaldéhyde, le 4-diméthylaminobenzaldéhyde, le 4-méthyl-5-imidazolecarboxaldéhyde, le 4-diméthylaminocinnamaldéhyde, le 4-hydroxy-2-méthoxybenzaldéhyde, le 3,5-diméthyl-4-hydroxybenzaldéhyde, le 4-diméthylamino-2-méthoxybenzaldéhyde, le 2-hydroxybenzaldéhyde, le 4-hydroxy-1-naphtaldéhyde, le 4-méthoxy-1-naphtaldéhyde, le 4-diméthylamino-1-naphtaldéhyde, le 4'-hydroxybiphényl-1-carbaldéhyde, le 2-hydroxy-3-méthoxybenzaldéhyde, le 2,4-dihydroxybenzaldéhyde, le 3,4-dihydroxybenzaldéhyde, le 2,5-dihydroxybenzaldéhyde, le 2,3,4-trihydroxybenzaldéhyde, le 3,4,5-trihydroxybenzaldéhyde, le 2,4,6-trihydroxybenzaldéhyde, le 2,4-diméthoxybenzaldéhyde, le 2,3-diméthoxybenzaldéhyde, le 2,5-diméthoxybenzaldéhyde, le 3,5-diméthoxybenzaldéhyde, le 3,4-diméthoxybenzaldéhyde, l'indole-3-carbaldéhyde, le benzène-1,4-dicarbaldéhyde, le 4-éthoxybenzaldéhyde, la 2-méthyl-1,4-naphtoquinone, le 4-carboxybenzaldéhyde, le 4-hydroxy-3-méthoxycinnamaldéhyde, le 3,5-diméthoxy-4-hydroxycinnamaldéhyde, le 3-méthoxy-4-(1-pyrrolidinyl)benzaldéhyde, le 4-diéthylamino-3-méthoxybenzaldéhyde, le 1,2-phtaldialdéhyde, le pyrrole-2-aldéhyde, le thiophène-2-aldéhyde, le thiophène-3-aldéhyde, le chromone-3-carboxaldéhyde, le 6-méthyl-4-oxo-1(4H)-benzopyranne-3-carbaldéhyde, le N-méthylpyrrole-2-aldéhyde, le 5-méthylfurfural, le 6-hydroxychromène-3-carboxaldéhyde, le 6-méthylindole-3-carboxaldéhyde, le 4-dibutylaminobenzaldéhyde, le N-éthylcarbazole-3-aldéhyde, le 4-diéthylamino-2-hydroxybenzaldéhyde, le 3,4-diméthoxy-5-hydroxybenzaldéhyde, le 5-(4-(diéthylamino)phényl)-2,4-pentadiénal, le 2,3-thiophènedicarboxaldéhyde, le 2,5-thiophènedicarboxaldéhyde, le 2-méthoxy-1-naphtaldéhyde, le 3-éthoxy-4-hydroxybenzaldéhyde, le 2-nitrobenzaldéhyde, le 3-nitrobenzaldéhyde et le 4-nitrobenzaldéhyde.

5. Composition selon l'une quelconque des revendications 1 à 4, **caractérisée en ce que** l'amine primaire est choisie parmi des alcanolamines, des amines aromatiques et des acides aminés.

6. Composition selon la revendication 5, **caractérisée en ce que** l'amine primaire est choisie parmi la monoéthanolamine, l'isopropanolamine, le 1-amino-2-propanol, le 3-amino-1-propanol et l'arginine.

7. Composition selon l'une quelconque des revendications 1 à 6, **caractérisée en ce que** le composant (A1) a un pH de 1 à 4,5.

8. Composition selon l'une quelconque des revendications 1 à 7, **caractérisée en ce que** le composant (A2) a un pH de 7,5 à 12.

9. Composition selon l'une quelconque des revendications 1 à 8, **caractérisée en ce qu'**elle contient les composés de formules (I) à (IX), les composés carbonyle et les amines, par rapport à la préparation prête à l'emploi, chacun en une quantité totale de 0,01 à 10 % en poids.

10. Procédé pour la coloration temporaire de fibres, en particulier de cheveux, dans lequel la fibre est colorée avec une composition selon l'une quelconque des revendications 1 à 9 et, à un instant ultérieur quelconque, de nouveau décolorée, **caractérisé en ce que**, pour la décoloration, on laisse agir sur la fibre colorée une préparation contenant un sulfite, pendant une durée de 5 à 60 minutes, à une température de 20 à 50°C.

11. Nécessaire à plusieurs composants, constitué d'un composant (A1) contenant une énamine de formule (I) et d'un composant (A2) contenant un composé carbonyle et une amine primaire.
